# EUROPEAN PATENT SPECIFICATION

(11) **EP 2 041 285 B1**
(45) Date of publication and mention of the grant of the patent: **12.10.2011**
(21) Application number: 07733352.4
(22) Date of filing: 25.06.2007
(51) Int. Cl.: C12N 15/53, C12N 9/02, C12N 1/21, C10G 32/00

(54) **Nucleic acid molecules encoding an enzyme that degrades long-chain n-alkenes**
Für ein langkettiger n-Alkan abbauendes Enzym kodierende Nukleinsäuremolekule
Acides nucléiques codant pour une enzyme capable de dégrader des n-alkanes à longue chaîne

(30) Priority: 23.06.2006 GB 0612538
(43) Date of publication of application: 01.04.2009
(73) Proprietor: Statoil ASA, 4035 Stavanger (NO)
(72) Inventor: KOTLAR, Hans, Kristian, 4035 Stavanger (NO); ELLINGSEN, Trond, 7465 Trondheim (NO); ZOTCHEV, Sergey, 7491 Trondheim (NO); WENTZEL, Alexander, 7491 Trondheim (NO); THRONE-HOLST, Mimmi, 7465 Trondheim (NO)
(74) Representative: Icely, Dominic Michael
(86) International application number: PCT/GB2007/002357
(87) International publication number: WO 2007/148121

(56) References cited:
- DATABASE Geneseq [Online] 20 November 2003 (2003-11-20), "Acinetobacter baumannii protein #165." XP002450713 retrieved from EBI accession no. GSP:ADA33004 Database accession no. ADA33004
- DATABASE Geneseq [Online] 20 November 2003 (2003-11-20), "DNA encoding Acinetobacter baumannii protein #165." XP002450714 retrieved from EBI accession no. GSN:ADA28878 Database accession no. ADA28878
- TANI AKIO ET AL: "Two acyl-CoA dehydrogenases of Acinetobacter sp. strain M-1 that uses very long-chain n-alkanes." JOURNAL OF BIOSCIENCE AND BIOENGINEERING, vol. 94, no. 4, 2002, pages 326-329, XP002450665 ISSN: 1389-1723
- THRONE-HOLST MIMMI ET AL: "Utilization of n-alkanes by a newly isolated strain of Acinetobacter venetianus: the role of two AlkB-type alkane hydroxylases" APPLIED MICROBIOLOGY AND BIOTECHNOLOGY, vol. 72, no. 2, 7 March 2006 (2006-03-07), pages 353-360, XP002450666 ISSN: 0175-7598
- KOKORIS MARK S ET AL: "Characterization of herpes simplex virus type 1 thymidine kinase mutants engineered for improved ganciclovir or acyclovir activity" PROTEIN SCIENCE, vol. 11, no. 9, September 2002 (2002-09), pages 2267-2272, XP002458318 ISSN: 0961-8368
- THRONE-HOLST MIMMI ET AL: "Identification of novel genes involved in long-chain n-alkane degradation by Acinetobacter sp. strain DSM 17874." APPLIED AND ENVIRONMENTAL MICROBIOLOGY MAY 2007, vol. 73, no. 10, May 2007 (2007-05), pages 3327-3332, XP002450667 ISSN: 0099-2240 cited in the application

### SEQUENCE LISTING

<110> Statoil ASA
<120> Nucleic Acid Molecules
<130> 1.91614/01
<150> GB0612538.9
   <151> 2006-06-23
<160> 24
<170> PatentIn version 3.3
<210> 1
   <211> 1491
   <212> DNA
   <213> Acinetobacter sp.
<400> 1
   atggaaaagc aagttgatgt attgattatt ggtgcaggta tctctgggat tgggttagct      60
   gtacatctct ctaaaaattg tccacaacgc aaatttgaaa tcttagagcg tcgtgaaagc     120
   tttggtggaa cttgggattt attccgctat cctggtattc gttcagattc tgatatgtca     180
   acgtttggtt ttaacttcaa gccatgggca aaagacaaag tgttggctag cggtgctgag     240
   atcaaaggct atttaagtga tgtgattagt gaaaatcagt taaaagacaa aattcacttt     300
   ggtcatcgtg tactttctgc gaactatgat tctacaaaga aaaaatggtt agttgagatc     360
   gaagataaca acaagaaaaa acaaacgtgg tctgcaaact tcgtaatggg ttgtacaggg     420
   tactataact acgatcaagg ttatgcacct aagttcccga aacaagaaga tttcaaaggt     480
   cagtttattc atccacagca ctggccagaa aatttagatt acacaggtaa aaaagttgtg     540
   atcatcggca gtggtgcaac tgcaattacc cttgtacctt ctatggtcaa aggtggtgca     600
   ggtcatgtga cgatgttaca acgttcgcca acgtatattg cgacgattcc ttctatcgac     660
   tttatttatg aaaaaacacg taaatttatg tctgaagaaa cggcttataa atttactcgt     720
   gcacgtaata ttggtatgca acgtggtatt tatgcgctag cgcaaaagta tccaaaaact     780
   gtacgtcgtt tattgttgaa aggcattgag ttgcagttga aaggcaaagt ggatatgaaa     840
   cactttactc caagttacaa tccttgggat caacgtttat gtgttgtgcc agatggtgat     900
   ttattcaaag cattgcgtga aggtcaggca agtgttgaga cagatcaaat cgagaaattc     960
   accgcaaatg gtattcaatt gaaatctggt aaacatttag aggctgatat tgtgatttca    1020
   gcaactggtt tagagattca gattttaggt ggtgtccaag gttcaatcga tggtaaacca    1080
   atgaatacat ctcaacatat gctttatcaa ggcgttatgg tgagcgatgt gcctaatatg    1140
   gcaatgatca ttggttatat caatgcgtca tggactttga aggttgatat tgctgctgat    1200
   tatatttgcc gtttgattaa tcatatggat aaaaatggtt ttgatgaggt gattgcacac    1260
   gccgatcctt cacagcgcga aaatgacacc atcatgggta aaatgtcatc tggttatatc    1320
   gcccgcgcag cggatgtgat gccaaaacaa ggtaagcaag caccttggaa gatcacaaat    1380
   aattaccttg cagatcgtaa ggagttaaaa gatgctaagt ttaatgatgg tgttttagaa    1440
   ttccataagc gcggtgagca aacagcaaac cgtaagccaa aattagtatc a              1491
<210> 2
   <211> 497
   <212> PRT
   <213> Acinetobacter sp.
<400> 2
   Met Glu Lys Gln Val Asp Val Leu Ile Ile Gly Ala Gly Ile Ser Gly
   1               5                   10                  15
   Ile Gly Leu Ala Val His Leu Ser Lys Asn Cys Pro Gln Arg Lys Phe
               20                  25                  30
   Glu Ile Leu Glu Arg Arg Glu Ser Phe Gly Gly Thr Trp Asp Leu Phe
           35                  40                  45
   Arg Tyr Pro Gly Ile Arg Ser Asp Ser Asp Met Ser Thr Phe Gly Phe
       50                  55                  60
   Asn Phe Lys Pro Trp Ala Lys Asp Lys Val Leu Ala Ser Gly Ala Glu
   65                  70                  75                  80
   Ile Lys Gly Tyr Leu Ser Asp Val Ile Ser Glu Asn Gln Leu Lys Asp
                   85                  90                  95
   Lys Ile His Phe Gly His Arg Val Leu Ser Ala Asn Tyr Asp Ser Thr
               100                 105                 110
   Lys Lys Lys Trp Leu Val Glu Ile Glu Asp Asn Asn Lys Lys Lys Gln
           115                 120                 125
   Thr Trp Ser Ala Asn Phe Val Met Gly Cys Thr Gly Tyr Tyr Asn Tyr
       130                 135                 140
   Asp Gln Gly Tyr Ala Pro Lys Phe Pro Lys Gln Glu Asp Phe Lys Gly
   145                 150                 155                 160
   Gln Phe Ile His Pro Gln His Trp Pro Glu Asn Leu Asp Tyr Thr Gly
                   165                 170                 175
   Lys Lys Val Val Ile Ile Gly Ser Gly Ala Thr Ala Ile Thr Leu Val
               180                 185                 190
   Pro Ser Met Val Lys Gly Gly Ala Gly His Val Thr Met Leu Gln Arg
           195                 200                 205
   Ser Pro Thr Tyr Ile Ala Thr Ile Pro Ser Ile Asp Phe Ile Tyr Glu
       210                 215                 220
   Lys Thr Arg Lys Phe Met Ser Glu Glu Thr Ala Tyr Lys Phe Thr Arg
   225                 230                 235                 240
   Ala Arg Asn Ile Gly Met Gln Arg Gly Ile Tyr Ala Leu Ala Gln Lys
                   245                 250                 255
   Tyr Pro Lys Thr Val Arg Arg Leu Leu Leu Lys Gly Ile Glu Leu Gln
               260                 265                 270
   Leu Lys Gly Lys Val Asp Met Lys His Phe Thr Pro Ser Tyr Asn Pro
   275 280 285
   Trp Asp Gln Arg Leu Cys Val Val Pro Asp Gly Asp Leu Phe Lys Ala
   290 295 300
   Leu Arg Glu Gly Gln Ala Ser Val Glu Thr Asp Gln Ile Glu Lys Phe
   305 310 315 320
   Thr Ala Asn Gly Ile Gln Leu Lys Ser Gly Lys His Leu Glu Ala Asp
   325 330 335
   Ile Val Ile Ser Ala Thr Gly Leu Glu Ile Gln Ile Leu Gly Gly Val
   340 345 350
   Gln Gly Ser Ile Asp Gly Lys Pro Met Asn Thr Ser Gln His Met Leu
   355 360 365
   Tyr Gln Gly Val Met Val Ser Asp Val Pro Asn Met Ala Met Ile Ile
   370 375 380
   Gly Tyr Ile Asn Ala Ser Trp Thr Leu Lys Val Asp Ile Ala Ala Asp
   385 390 395 400
   Tyr Ile Cys Arg Leu Ile Asn His Met Asp Lys Asn Gly Phe Asp Glu
   405 410 415
   Val Ile Ala His Ala Asp Pro Ser Gln Arg Glu Asn Asp Thr Ile Met
   420 425 430
   Gly Lys Met Ser Ser Gly Tyr Ile Ala Arg Ala Ala Asp Val Met Pro
   435 440 445
   Lys Gln Gly Lys Gln Ala Pro Trp Lys Ile Thr Asn Asn Tyr Leu Ala
   450 455 460
   Asp Arg Lys Glu Leu Lys Asp Ala Lys Phe Asn Asp Gly Val Leu Glu
   465 470 475 480
   Phe His Lys Arg Gly Glu Gln Thr Ala Asn Arg Lys Pro Lys Leu Val
   485 490 495
   Ser
<210> 3
   <211> 498
   <212> DNA
   <213> Acinetobacter sp.
<400> 3
   atgagaaaaa tcatcatttt ggggactatc ttacttagta cattcagtta tgctgatgat     60
   gttaaacaaa agacagcaat tgctgagaag ttggtttcgg ttgatggtac tgaacaagga    120
   cttgaaaata cggataaaat gattcttgaa caaattcgta tgcgcttacc aaaagatatg    180
   ccagctaatt ttttcactga tttaactaaa aacttaaata gtgaacaacg taaacaattt    240
   attgttcagc gttatgtgga aacgtttagc cagaaagagt tacaagccgc tttaaacttt    300
   tatcagtcaa atgaagggaa ggcgtgggca aagaaagcaa gtgaaattgg aggagaggtt    360
   gctcatttca caacgcaaaa tgctcgtaat gctttaaaca ctaccatgca agaacatgtt    420
   gagaatccta ccgtaaaaca gttaatggct cgtatgaacc cagcaccagt agctgcggta    480
   gaaaaagctg aacagaaa                                                  498
<210> 4
   <211> 166
   <212> PRT
   <213> Acinetobacter sp.
<400> 4
   Met Arg Lys Ile Ile Ile Leu Gly Thr Ile Leu Leu Ser Thr Phe Ser
   1               5                   10                  15
   Tyr Ala Asp Asp Val Lys Gln Lys Thr Ala Ile Ala Glu Lys Leu Val
               20                  25                  30
   Ser Val Asp Gly Thr Glu Gln Gly Leu Glu Asn Thr Asp Lys Met Ile
           35                  40                  45
   Leu Glu Gln Ile Arg Met Arg Leu Pro Lys Asp Met Pro Ala Asn Phe
       50                  55                  60
   Phe Thr Asp Leu Thr Lys Asn Leu Asn Ser Glu Gln Arg Lys Gln Phe
   65                  70                  75                  80
   Ile Val Gln Arg Tyr Val Glu Thr Phe Ser Gln Lys Glu Leu Gln Ala
                   85                  90                  95
   Ala Leu Asn Phe Tyr Gln Ser Asn Glu Gly Lys Ala Trp Ala Lys Lys
               100                 105                 110
   Ala Ser Glu Ile Gly Gly Glu Val Ala His Phe Thr Thr Gln Asn Ala
           115                 120                 125
   Arg Asn Ala Leu Asn Thr Thr Met Gln Glu His Val Glu Asn Pro Thr
       130                 135                 140
   Val Lys Gln Leu Met Ala Arg Met Asn Pro Ala Pro Val Ala Ala Val
   145                 150                 155                 160
   Glu Lys Ala Glu Gln Lys
                   165
<210> 5
   <211> 2272
   <212> DNA
   <213> Acinetobacter sp.
<400> 5
   gacatgtgta ttgtcaaatt tgtgctttaa taaagtcatg gtttatacag ggtataaaaa      60
   aaatggaaaa gcaagttgat gtattgatta ttggtgcagg tatctctggg attgggttag     120
   ctgtacatct ctctaaaaat tgtccacaac gcaaatttga aatcttagag cgtcgtgaaa     180
   gctttggtgg aacttgggat ttattccgct atcctggtat tcgttcagat tctgatatgt     240
   caacgtttgg ttttaacttc aagccatggg caaaagacaa agtgttggct agcggtgctg     300
   agatcaaagg ctatttaagt gatgtgatta gtgaaaatca gttaaaagac aaaattcact     360
   ttggtcatcg tgtactttct gcgaactatg attctacaaa gaaaaaatgg ttagttgaga     420
   tcgaagataa caacaagaaa aaacaaacgt ggtctgcaaa cttcgtaatg ggttgtacag     480
   ggtactataa ctacgatcaa ggttatgcac ctaagttccc gaaacaagaa gatttcaaag     540
   gtcagtttat tcatccacag cactggccag aaaatttaga ttacacaggt aaaaaagttg     600
   tgatcatcgg cagtggtgca actgcaatta cccttgtacc ttctatggtc aaaggtggtg     660
   caggtcatgt gacgatgtta caacgttcgc caacgtatat tgcgacgatt ccttctatcg     720
   actttattta tgaaaaaaca cgtaaattta tgtctgaaga aacggcttat aaatttactc     780
   gtgcacgtaa tattggtatg caacgtggta tttatgcgct agcgcaaaag tatccaaaaa     840
   ctgtacgtcg tttattgttg aaaggcattg agttgcagtt gaaaggcaaa gtggatatga     900
   aacactttac tccaagttac aatccttggg atcaacgttt atgtgttgtg ccagatggtg     960
   atttattcaa agcattgcgt gaaggtcagg caagtgttga gacagatcaa atcgagaaat    1020
   tcaccgcaaa tggtattcaa ttgaaatctg gtaaacattt agaggctgat attgtgattt    1080
   cagcaactgg tttagagatt cagattttag gtggtgtcca aggttcaatc gatggtaaac    1140
   caatgaatac atctcaacat atgctttatc aaggcgttat ggtgagcgat gtgcctaata    1200
   tggcaatgat cattggttat atcaatgcgt catggacttt gaaggttgat attgctgctg    1260
   attatatttg ccgtttgatt aatcatatgg ataaaaatgg ttttgatgag gtgattgcac    1320
   acgccgatcc ttcacagcgc gaaaatgaca ccatcatggg taaaatgtca tctggttata    1380
   tcgcccgcgc agcggatgtg atgccaaaac aaggtaagca agcaccttgg aagatcacaa    1440
   ataattacct tgcagatcgt aaggagttaa aagatgctaa gtttaatgat ggtgttttag    1500
   aattccataa gcgcggtgag caaacagcaa accgtaagcc aaaattagta tcataatttt    1560
   tgcttgaata atttgaaacc ctatgcatgg catagggttt tttatttgtg ctcagggttt    1620
   aaatcttaga aatatcgcgt atattgagca cattagaaaa tttatattag ggataatacg    1680
   atgagaaaaa tcatcatttt ggggactatc ttacttagta cattcagtta tgctgatgat    1740
   gttaaacaaa agacagcaat tgctgagaag ttggtttcgg ttgatggtac tgaacaagga    1800
   cttgaaaata cggataaaat gattcttgaa caaattcgta tgcgcttacc aaaagatatg    1860
   ccagctaatt ttttcactga tttaactaaa aacttaaata gtgaacaacg taaacaattt    1920
   attgttcagc gttatgtgga aacgtttagc cagaaagagt tacaagccgc tttaaacttt    1980
   tatcagtcaa atgaagggaa ggcgtgggca aagaaagcaa gtgaaattgg aggagaggtt    2040
   gctcatttca caacgcaaaa tgctcgtaat gctttaaaca ctaccatgca agaacatgtt    2100
   gagaatccta ccgtaaaaca gttaatggct cgtatgaacc cagcaccagt agctgcggta    2160
   gaaaaagctg aacagaaata atcaatcaaa aagggatcat aatgatgatc ccttttttat    2220
   gctatttatt tttatagagt acgagaaata agttcttcca tgatctcatt gg            2272
<210> 6
   <211> 28
   <212> DNA
   <213> Artificial
<220>
   <223> oligonucleotide Kan1
<400> 6
   gctctagacc gtcaagtcag cgtaatgc 28
<210> 7
   <211> 20
   <212> DNA
   <213> Artificial
<220>
   <223> oligonucleotide Tn10:1
<400> 7
   ggatcatatg acaagatgtg 20
<210> 8
   <211> 20
   <212> DNA
   <213> Artificial
<220>
   <223> oligonucleotide AVM1004A7-1
<400> 8
   accctgtaca acccattacg 20
<210> 9
   <211> 20
   <212> DNA
   <213> Artificial
<220>
   <223> oligonucleotide AVM1004A7-2,
<400> 9
   agttgtgatc atcggcagtg 20
<210> 10
   <211> 28
   <212> DNA
   <213> Artificial
<220>
   <223> oligonucleotide AVM1004A7-3
<400> 10
   gctctagact atcctggtat tcgttcag 28
<210> 11
   <211> 28
   <212> DNA
   <213> Artificial
<220>
   <223> oligonucleotide AVM1004A7-4
<400> 11
   cgggatccta aataccacgt tgcatacc 28
<210> 12
   <211> 31
   <212> DNA
   <213> Artificial
<220>
   <223> oligonucleotide AVMext2
<400> 12
   caaggtaatg cattggcttg ggctacctca g 31
<210> 13
   <211> 31
   <212> DNA
   <213> Artificial
<220>
   <223> oligonucleotide AVMext1
<400> 13
   ttctatgaaa aagaagttgt tccaaatatt g 31
<210> 14
   <211> 25
   <212> DNA
   <213> Artificial
<220>
   <223> oligonucleotide AVM1004A7-5
<400> 14
   cgtttatgtg ttgtgccaga tggtg 25
<210> 15
   <211> 28
   <212> DNA
   <213> Artificial
<220>
   <223> oligonucleotide AVMext3
<400> 15
   gaaatatcgc gtatattgag cacattag 28
<210> 16
   <211> 21
   <212> DNA
   <213> Artificial
<220>
   <223> oligonucleotide AVMext4
<400> 16
   attgtcgata tatgcgcgtg c 21
<210> 17
   <211> 25
   <212> DNA
   <213> Artificial
<220>
   <223> oligonucleotide alm-prom-up
<400> 17
   gacatgtgta ttgtcaaatt tgtgc 25
<210> 18
   <211> 21
   <212> DNA
   <213> Artificial
<220>
   <223> oligonucleotide almB-end-lo
<400> 18
   ccaatgagat catggaagaa c 21
<210> 19
   <211> 42
   <212> DNA
   <213> Artificial
<220>
   <223> oligonucleotide fuf1x1.1
<400> 19
   gtatgcgctt accaaaagat atgaacccag caccagtagc tg 42
<210> 20
   <211> 26
   <212> DNA
   <213> Artificial
<220>
   <223> oligonucleotide AVM1004A7-6
<400> 20
   gagagatgta cagctaaccc aatccc 26
<210> 21
   <211> 36
   <212> DNA
   <213> Artificial
<220>
   <223> oligonucleotide AVM1004A7-7Sph
<400> 21
   gcgcgcgcat gcaatcatca ttgccatatt aggcac 36
<210> 22
   <211> 42
   <212> DNA
   <213> Artificial
<220>
   <223> oligonucleotide fuf1x1.2
<400> 22
   cagctactgg tgctgggttc atatcttttg gtaagcgcat ac 42
<210> 23
   <211> 496
   <212>. PRT
   <213> Acinetobacter sp.
<400> 23
   Met Glu Lys Gln Val Asp Val Leu Ile Ile Gly Ala Gly Ile Ser Gly
   1 5 10 15
   Ile Gly Leu Ala Val His Leu Ser Lys Asn Cys Pro Gln Arg Lys Phe
   20 25 30
   Glu Ile Leu Glu Arg Arg Asp Ser Phe Gly Gly Thr Trp Asp Leu Phe
   35 40 45
   Arg Tyr Pro Gly Ile Arg Ser Asp Ser Asp Met Ser Thr Phe Gly Phe
   50 55 60
   Asn Phe Lys Pro Trp Ala Lys Asp Lys Val Leu Ala Ser Gly Ala Glu
   65 70 75 80
   Ile Lys Gly Tyr Leu Ser Asp Val Ile Ser Glu Asn Gln Leu Lys Asp
   85 90 95
   Lys Ile His Phe Gly His Arg Val Leu Ser Ala Asn Tyr Asp Ser Ala
   100 105 110
   Lys Lys Lys Trp Ala Val Glu Ile Glu Asp Ser Asn Lys Lys Lys Gln
   115 120 125
   Thr Trp Ser Ala Asn Phe Val Met Gly Cys Thr Gly Tyr Tyr Asn Tyr
   130 135 140
   Asp Gln Gly Tyr Ala Pro Lys Phe Pro Lys Gln Glu Asp Phe Lys Gly
   145 150 155 160
   Gln Leu Ile His Pro Gln His Trp Pro Glu Asn Leu Asp Tyr Thr Gly
   165 170 175
   Lys Lys Val Val Ile Ile Gly Ser Gly Ala Thr Ala Ile Thr Leu Val
   180 185 190
   Pro Ser Met Val Lys Gly Gly Ala Gly His Val Thr Met Leu Gln Arg
   195 200 205
   Ser Pro Ser Tyr Ile Ala Thr Ile Pro Ser Ile Asp Phe Ile Tyr Glu
   210 215 220
   Lys Thr Arg Lys Phe Met Ser Glu Glu Thr Ala Tyr Lys Phe Thr Arg
   225 230 235 240
   Ala Arg Asn Ile Gly Met Gln Arg Gly Ile Tyr Ala Leu Ala Gln Lys
   245 250 255
   Tyr Pro Lys Thr Val Arg Arg Leu Leu Leu Lys Gly Ile Glu Leu Gln
   260 265 270
   Leu Lys Gly Lys Val Asp Met Lys His Phe Thr Pro Ser Tyr Asn Pro
   275 280 285
   Trp Asp Gln Arg Leu Cys Val Val Pro Asp Gly Asp Leu Phe Lys Ala
   290 295 300
   Leu Arg Glu Gly Gln Ala Asn Val Glu Thr Asp Gln Ile Glu Lys Phe
   305 310 315 320
   Thr Ala Asn Gly Ile Gln Leu Lys Ser Gly Lys His Leu Glu Ala Asp
                   325                 330                 335
   Ile Val Ile Ser Ala Thr Gly Leu Glu Ile Gln Ile Leu Gly Gly Val
               340                 345                 350
   Lys Gly Thr Ile Asp Gly Lys Pro Met Asp Thr Ser Gln His Met Leu
           355                 360                 365
   Tyr Gln Gly Val Met Val Ser Asp Val Pro Asn Met Ala Met Ile Ile
       370                 375                 380
   Gly Tyr Ile Asn Ala Ser Trp Thr Leu Lys Val Asp Ile Ala Ala Asp
   385                 390                 395                 400
   Tyr Ile Cys Arg Leu Leu Asn His Met Asp Lys Asn Gly Tyr Asp Glu
                   405                 410                 415
   Val Ile Ala His Ala Asp Pro Ala Leu Arg Glu Gln Asp Thr Ile Met
               420                 425                 430
   Gly Lys Met Ser Ser Gly Tyr Ile Ala Arg Ala Ala Asp Val Met Pro
           435                 440                 445
   Lys Gln Gly Lys Lys Ala Pro Trp Lys Ile Thr Asn Asn Tyr Leu Ala
       450                 455                 460
   Asp Arg Lys Glu Leu Lys Asp Ala Ser Phe Asn Asp Val Val Leu Gln
   465                 470                 475                 480
   Phe His Lys Arg Gly Glu Gln Val Asp Arg Lys Pro Lys Leu Val Ser
                   485                 490                 495
<210> 24
   <211> 1491
   <212> DNA
   <213> Acinetobacter sp.
<400> 24
   atggaaaagc aagttgatgt attaattatt ggtgcaggta tctctgggat tgggctagct     60
   gtgcatctct caaagaactg tccacaacgt aaattcgaaa tcttagaacg tcgtgatagc    120
   tttggtggga cttgggattt attccgttac cctggtattc gttctgactc ggatatgtca    180
   acatttggtt ttaattttaa accatgggca aaagacaaag tattggcaag cggtgctgag    240
   atcaagggct atttaagcga tgtgatcagt gaaaatcagt taaaagataa aattcatttt    300
   ggtcatcgtg tcctttctgc aaactacgat tctgcaaaga aaaaatgggc tgttgaaatt    360
   gaagacagta acaagaaaaa gcaaacttgg tctgcaaatt ttgtgatggg ctgtacaggc    420
   tactacaact atgatcaagg ttatgcaccg aaattcccaa aacaagaaga tttcaaaggc    480
   caattgatcc acccacagca ctggcctgaa aacctagatt atacaggcaa gaaagtggtg    540
   atcattggta gtggtgcaac agcaattacc cttgttcctt caatggtgaa aggcggtgca    600
   gggcatgtga ctatgttaca gcgttctcct tcttatattg cgacgattcc ttcaattgac    660
   ttcatttatg aaaaaacgcg taaatttatg tctgaggaaa ccgcttataa atttacccgt    720
   gcacgtaata ttggtatgca acgtggtatt tatgcattag cacaaaaata tccaaaaaca    780
   gtacgtcgct tattgttgaa aggtattgag ttgcagttga aaggtaaggt ggatatgaaa    840
   cactttacac caagctataa cccttgggat caacgtttat gtgtcgtacc agatggtgat    900
   ttattcaagg cattacgtga aggccaagcc aatgttgaaa cagatcaaat tgaaaaattc    960
   acggcaaatg gtattcaatt gaagtctggt aaacaccttg aagcagatat tgtgatttca   1020
   gcaacaggtt tagaaatcca aatcttgggt ggcgtgaaag gcacaattga tggtaagcca   1080
   atggatacct ctcaacacat gttatatcaa ggtgtgatgg tcagtgatgt accaaatatg   1140
   gcaatgatca ttggctatat caatgcatct tggaccttaa aggttgatat tgcagcggat   1200
   tatatctgtc gtttgctgaa tcatatggac aaaaatggct atgatgaagt cattgcccat   1260
   gcggatccag cattgcgtga acaagatacc attatgggta aaatgtcttc tggttatatt   1320
   gcacgtgcag cagatgtaat gccgaagcaa ggtaaaaaag caccgtggaa aatcaccaat   1380
   aactatcttg cggatcgtaa agaattgaaa gatgcatcat tcaatgacgt ggtcctgcaa   1440
   ttccataaac gtggtgagca agtcgatcgt aagccaaagc tagtgtcgta a             1491

## Description

The present invention relates to nucleic acid molecules that encode enzymes, more especially putative monoxygenases, involved in the metabolism of solid crude oil components, the encoded enzymes, bacteria containing such nucleic acid molecules and the encoded enzymes and methods of using such bacteria and enzymes to cause biodegradation of waxes present in oil, in particular to lower the pour point of oil, e.g. during industrial processes.

In order to optimise oil recovery during the industrial processes of oil production, it is advantageous that the viscosity of the oil be reduced so that oil is able to flow readily. When oil can flow, it can be pumped and moved more easily, and extracted from geological formations more easily. Reducing the viscosity of oil will also assist in removal of any unwanted oil from land when it is present as a contaminant. It is, however, difficult to achieve this reduction in viscosity, particularly in cold regions such as the Arctic and Antarctic, and in regions that produce oil having an *n-*alkane content which causes the pour point of the oil to be high, such as in Azerbaijan.

Various techniques are currently used to try to overcome these problems, such as the use of wax deposition inhibitors, or the use of heated pipes for transporting crude oil. These presently employed methods are, however, expensive to carry out and logistically complicated to put into place.

As mentioned above, it is particularly important to try to counteract the effects which arise from the high long-chain *n*-alkane content of crude oil, particularly in regions where the oil is naturally rich in long-chain *n*-alkanes. The melting temperature of longer chain alkanes is higher than that of shorter-chain alkanes and they form solids at higher temperatures. C10-C20 *n*-alkanes are in general liquid, but this will also depend on the particular conditions to which these alkanes are exposed e.g. the temperatures to which they are exposed

Instead of having to put into place remedial methods to deal with the damaging effects of high *n*-alkane content, it may be possible to circumvent some or all of the problems by degradation of long-chain *n*-alkanes, e.g. in crude oil. Esterification of n-alkanes, which can also occur during degradation also affects the viscosity of oil (and has the further advantage of increasing the fuel yield of the oil). It is also known that the conversion of petroleum compounds into activated intermediates e.g. for the polymer and bulk chemical industries is attractive, because the functionalization of hydrocarbons by chemical means is difficult, energy intensive, and environmentally unfriendly. Indeed, new industrial synthetic routes for difficult-to-synthesize complex molecules, such as secondary metabolites (steroids, polyketides, terpenes, etc.) and pharmaceutical and agrochemical intermediates would be desirable.

Aerobic and non-aerobic n-alkane degradation is a widespread phenomenon in nature, and several microbial strains and enzymes involved in aerobic n-alkane degradation have been isolated and studied in some detail (reviewed in van Beilen and Funhoff, 2005, Curr Opin Biotechnol, 16(3): 308-14), e.g. cytochrome P450, monooxygenase and dioxygenase. Short-chain *n*-alkane degrading enzymes in particular have been characterised, and naturally occurring enzymes thus exist which have the potential to be used to degrade such short-chain *n*-alkanes.

Alkane molecules are chemically inert and must be activated to allow further metabolic steps. One aerobic degradation pathway for short to medium-chain alkanes has been well studied. In this pathway a membrane bound monooxygenase AlkB (which is also known as AlkM) initially activates alkanes by oxidising them to form the corresponding primary alcohols. Further steps (carried out by alcohol dehydrogenases, aldehyde dehydrogenases and acyl CoA synthetase) cause the oxidation of the resultant alcohols and aldehydes to fatty acids and the entry of the fatty acids into the β oxidation pathway.

Although a number of *n*-alkane degrading enzymes are known, these are primarily short-chain *n*-alkane degrading enzymes. Further *n*-alkane degrading enzymes (i.e. enzymes which catalyse one or more of the steps that are involved in *n*-alkane degradation, as defined herein) would be highly desirable, and in particular those that can degrade long-chain *n*-alkanes. In particular, enzymes that can degrade long-chain *n*-alkanes having 26 or more, 28 or more or 30 or more carbon atoms, would be very desirable, and enzymes and microorganisms containing such enzymes can be applied to the processes of oil recovery.

It is, however, difficult to identify such enzymes. In order to identify enzymes responsible for a certain phenotype, the combination of random transposon mutagenesis and elaborate selection strategies is an effective tool and has widely been used (reviewed by Hayes, 2003, Annual Review of Genetics 37: 3-29). Nevertheless, no specific selection method presently exists to identify enzymes involved in metabolism of solid crude oil components (e.g. long-chain *n*-alkanes). Methods of identifying long-chain *n*-alkane utilising bacteria and the particular enzymes within these bacteria which are responsible for this property are thus also desirable.

Tani et al. J. Biosci. Bioeng. (2002) 94, 326-329 discloses an acyl-CoA dehydrogenase (AcdB) of *Acinetobacter* M-I that degrades long chain alkanes (n above 20)

In work leading up to the invention, the inventors identified a further bacterial strain of the *Acinetobacter* sp. capable of utilizing *n*-alkanes with chain lengths ranging from decane (C₁₀H₂₂) to tetracontane (C₄₀H₈₂) as a sole carbon source. This strain was isolated using a system for screening for microorganisms able to grow on paraffin (mixed long-chain *n*-alkanes). The isolate, identified according to its 16S rRNA sequence as *Acinetobacter venetianus,* was designated *Acinetobacter* sp. DSM17874. Strain DSM17874 has been deposited at the DSMZ both in the open collection (deposit number DSM17874 by Mimmi Throne-Holst) and under the conditions of the Budapest Treaty on 20 June 2007, by Statoil ASA, under accession number DSM19446.

Using this strain, two *alkM* paralogues (*alkMa, alkMb*) were shown to have central roles in the utilization of C10 to C18 alkanes. This was achieved by making and analysing the growth of disruption mutants in *Acinetobacter* sp. DSM17874 (referred to in Throne-Holst *et al., 2006 as A. venetianus* 6A2) of the *alkMa, alkMb* genes, and a double mutant *alkMa*/*alkMb* (Throne-Holst et al., 2006 Appl Microbiol Biotechnol 72(2),353-360. However, since the double mutants and both of the single mutants were able to grow on *n*-alkanes with chain lengths of C20 and longer, it was concluded that at least one additional enzyme for utilization of these *n*-alkanes had to exist in *Acinetobacter* sp. DSM17874.

In order to identify the additional enzymes in *Acinetobacter* sp. DSM17874 that are responsible for utilizing *n*-alkanes with chain-lengths longer than C20, a novel high-throughput method has been developed. In this novel method, mutant strains of *Acinetobacter* sp. DSM17874 were generated and grown on plates with a long-chain alkane as a carbon source (e.g. a C32 *n*-alkane) applied as a powder. Viable clones were identified *in situ* by staining with iodonitrotetrazolium chloride (INT) and growth deficient clones were subjected to a step of counter-selection on e.g. acetate and short-chain alkanes. Only those clones that were deficient for growth on long-chain alkanes and which could grow on the alternative substrate were analyzed to identify the genes that were mutated in these clones, and that were hence required for growth on the long-chain *n*-alkane. This method is particularly useful as the parent strain can grow on both the C32 substrate and on the alternative substrate. As such, the presence of the counter selection step (i.e. the step of identifying clones that grow on the alternative substance but that can't grow on C32) eliminates from investigation any mutant strains which have mutations affecting pathways other than those required for growth on the C32 *n*-alkane substrate (i.e. mutations that affect cell viability generally). In other words, if a mutant strain can grow on the alternative substrate but not on the long-chain *n*-alkane substrate, yet the parent strain can grow on both, the mutated gene is likely to be of interest since it specifically affects the ability of the strain to grow on the long-chain (e.g. C32) *n-*alkane substrate.

The above method was used to identify the gene *almA,* which was shown to be essential for growth of *Acinetobacter* sp. DSM17874 on long-chain alkanes such as C32 and C36, but is not required for growth on other substrates (e.g. C16, C20 or C24). These features indicate that AlmA has a central role in the utilization of C32 and C36 *n*-alkanes.

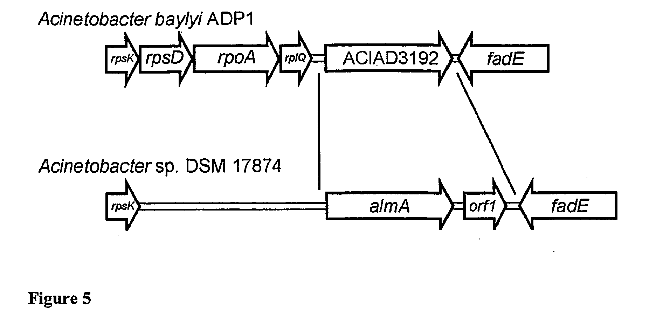

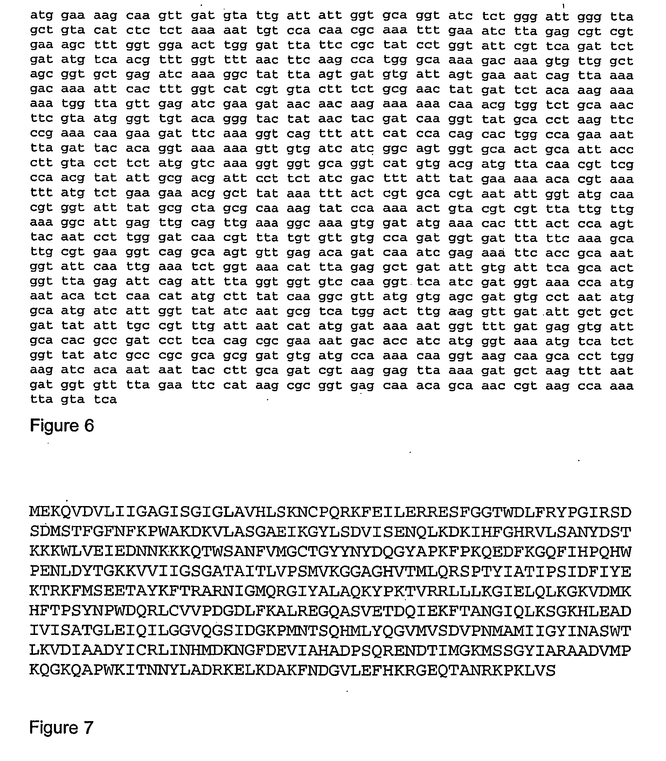

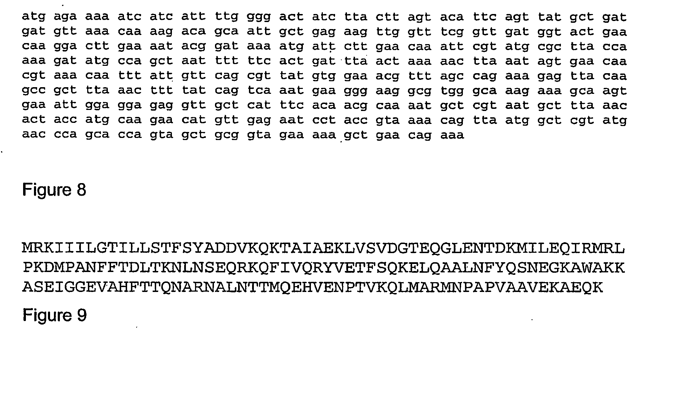

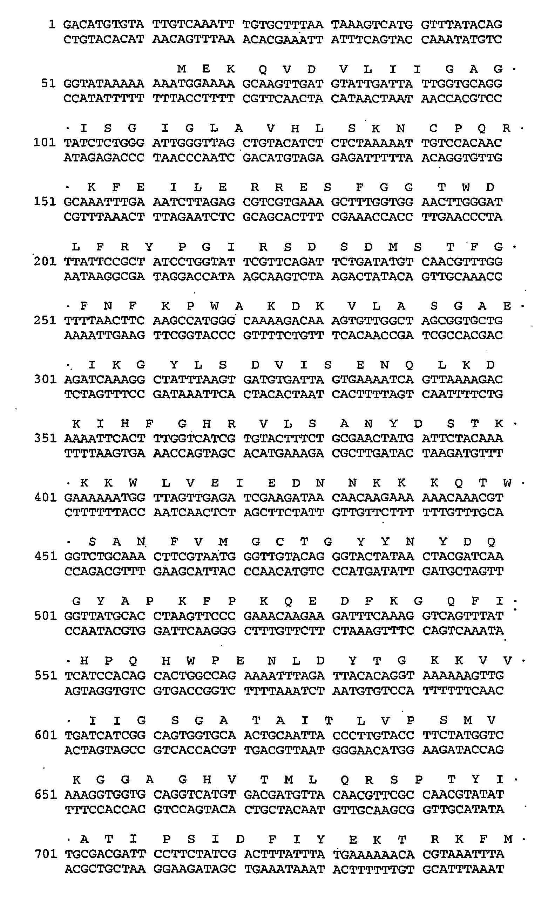

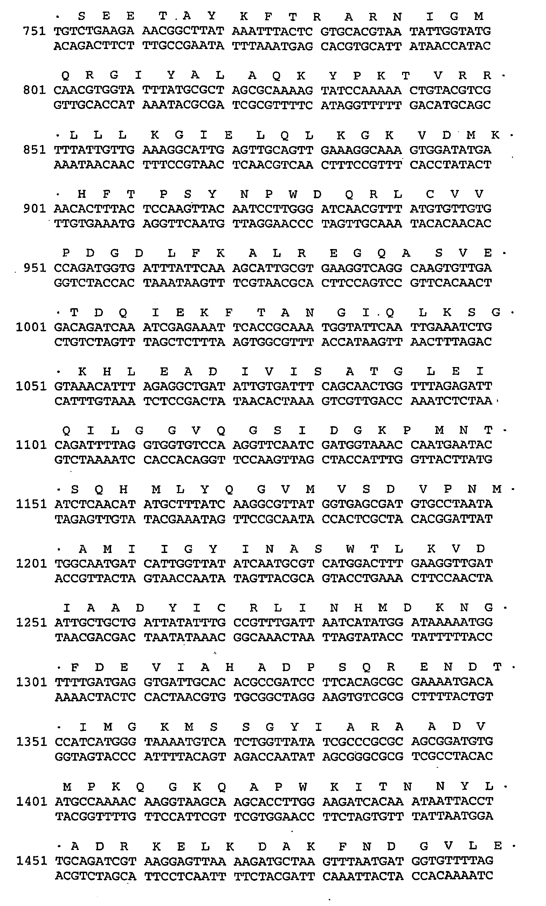

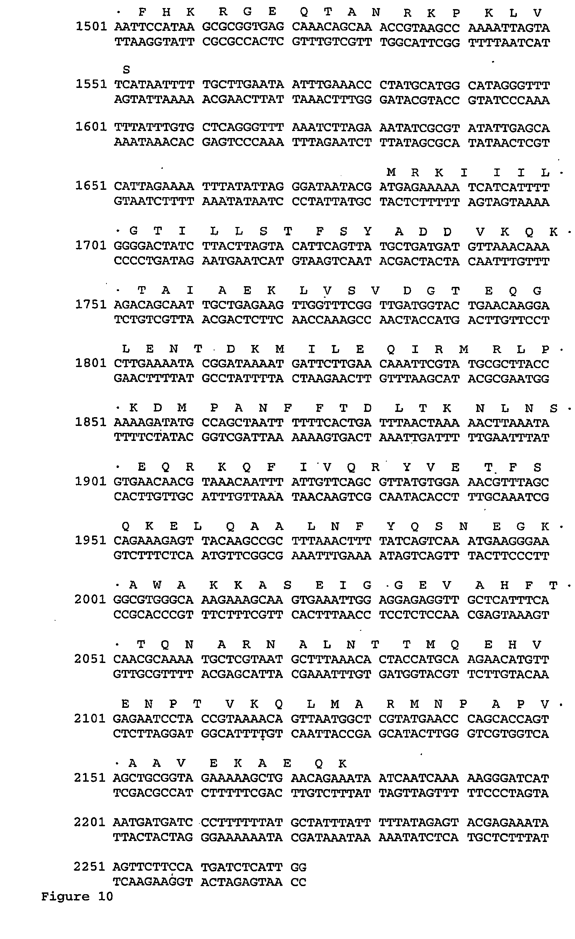

The nucleic acid sequence of the *almA* gene is set out in SEQ ID NO: (Figure 6), whilst the sequence of the encoded protein is represented by SEQ ID NO:2 (Figure 7). A further gene *orf1* (also referred to herein as *almB)* has also been identified based on its proximity to *almA* in the genome of *Acinetobacter* sp. DSM 17874. The nucleic acid sequence of the *orf1 (almB)* gene is set out in SEQ ID NO:3 (Figure 8), whilst the sequence of the encoded protein is represented by SEQ ID NO:4 (Figure 9). The *almA-orf1 (almAB)* locus is represented by SEQ ID NO:5 (Figure 10), and the similarity of organisation of this region with the region surrounding ACIAD3192 in *Acinetobacter* sp. ADP1 is shown in Figure 5.

The invention provides a nucleic acid molecule which encodes a putative monoxygenase protein that has a role in the degradation of an *n*-alkane substrate having 26 or more carbons, comprising a nucleic acid molecule selected from the group consisting of:
(i) a nucleic acid molecule whose nucleotide sequence is set forth in SEQ ID NO:1,
(ii) a nucleic acid molecule whose nucleotide sequence has at least 80% sequence identity, preferably at least 85, 90, 95, 98% and more preferably at least 99% sequence identity, with a nucleic acid molecule whose nucleotide sequence is set forth in SEQ ID NO:1,
(ii) a nucleic acid molecule encoding the protein whose amino acid sequence is set forth in SEQ ID NO:2,
(iv) a nucleic acid molecule whose nucleotide sequence has at least 85% sequence identity, preferably at least 90, 95, 98% and more preferably at least 99% sequence identity, with a nucleic acid molecule encoding the protein whose amino acid sequence is set forth in SEQ ID NO:2, and
(v) a nucleic acid molecule whose nucleotide sequence is complementary to the sequence of the nucleic acid molecule of any one of (i) to (iv).

Preferably, the nucleic acid molecule encodes a protein that has a role in the degradation of *n*-alkanes having 28 or more or 30 or more carbons (e.g. C30-C36).

The activity of the encoded protein can alternatively or additionally be defined in one or more of the following ways.

It has been shown that after exposure of a long-chain *n*-alkane containing substrate to the encoded protein, the production of alcohols, acids and/or esters can be detected. In some cases up to 70% of the substrate was seen to be esterified. This is advantageous since it increases the fuel yield of the substrate. As such, in one alternative, the activity of the encoded protein can be defined as causing the production of alcohols, acids and/or esters from an *n*-alkane, preferably from a long-chain *n*-alkane, more preferably a long-chain *n*-alkane having at least 26 or at least 30 carbon atoms. Such reactions may involve a reduction in *n*-alkane chain length, or the chain length may remain the same.

This activity can be measured by exposing an appropriate substrate to the encoded protein. This may be in the form of a purified protein, or by using a microorganism that is capable of providing said protein, or a cell extract of such a microorganism, for a time and under conditions which allow these reactions to occur. Suitable reaction conditions are e.g. as set out in Maeng et al., 1996 J. Bacteriol. 178 (13): 3695. For example, the substrate (e.g. C32 *n*-alkane, e.g. 100µM added to 100ml of 50mM Tris-HCl (pH7.5) containing 1mg of detergent, Plysurf A21OG (Daiichi Kogyo Seuyaky, Kyoto, Japan)) can be mixed with an appropriate amount of enzyme and incubated at approximately 30°C for 3 minutes with shaking.

It is possible to measure either the consumption of the substrate, or the production of the products, e.g. by gas chromatography or GC-MS. The consumption of the substrate, the generation of one or more product and/or the overall profile or fingerprint of the substrate can thus be evaluated in this way. For example the conversion of alkanes to the corresponding primary alcohols can be measured by GC and GC-MS as is carried out in Feng et al., 2007 PNAS 104:5602-5607, in which reaction mixtures containing 50 mM Tris-HCl (pH 7.5), 1 mM hexadecane (or other individual alkanes), 1 mM each of NADH and MgSO₄, and adequate amount of the enzyme to be tested were used. A mixture without enzyme was used as the control. The mixtures were incubated at 60°C with shaking for 5 min before extraction with cyclehexane, and alkane residues in the extract was analyzed by GC. Identification of the reaction product was carried out by GC-MS. Specific activity is expressed as units/mg enzyme. One unit of enzyme activity may be defined as the amount of enzyme that catalyses the consumption of 1.0µmol of substrate under these conditions. Preferably at least 5, 10, 15, 20, 25, 30, 35, 40, 50, 60, 70, 80, 90 or 95% of the substrate is consumed, or converted into product following such an incubation.

A suitable cell extract can be prepared by suspending washed cells in buffer (e.g. 50mM Tris-Cl) and disrupting the cells with sonication. The crude extract or a supernatant prepared from the extract can then be used as a source of enzyme.

In a further alternative, the activity of the encoded protein can be defined as being capable of lowering the pour point of an n-alkane-containing substrate such as crude oil.

Pour point is defined as the temperature at which a fluid ceases to pour, i.e. the lowest temperature (in °F or °C) at which a liquid remains pourable (meaning it still behaves as a fluid). As noted above, in oils, the pour point is generally increased by a high paraffin (or high long-chain n-alkane) content. High pour points usually occur in crude oils that have significant paraffin content. Paraffins (or waxes) will start to precipitate as temperature decreases. At some point the precipitates accumulate to the point where the fluid can no longer flow. The pour point for oil can be determined under protocols set forth in the ASTM D-97 pour point test, in which the pour point is established as that temperature at which oil ceases to flow when the sample is held at 90 degrees to the upright for five seconds.

It is advantageous to lower the pour point of a substrate such as crude oil, so that it becomes less viscous and hence easier to transport and recover. Even lowering the pour point of a substrate by a small amount can affect the ability of the substrate to flow and hence the ability to transport and recover the substrate.

As such, the pour point of a substrate containing n-alkanes can be measured before and after exposure of the substrate to a protein that is believed to share the activity of AlmA, for a period of time and under conditions that are sufficient to modify the pour point of said substrate, and if the pour point has been lowered, the protein is considered to share the same activity as the AlmA protein. Preferably the pour point is lowered by at least 0.1, 0.25, 0.5, 1, 1.5, 2, 3, 4, 5, 6, 7, 8, 9, 10, 15, 20 or more degrees.

The above tests can also be carried out in parallel with the exposure of the same substrate to the enzyme AlmA and the degree or amount of product formation or substrate consumption, or the change in pour point that is produced by exposure to a protein can be compared to that which is achieved with this enzyme. Preferably at least 30, 40, 50, 60, 70, 80 or 90% of the *n*-alkane substrate is consumed, as compared to the degree of consumption which is achieved following exposure of the same substrate to the protein encoded by the *almA* gene for the same time under the same conditions. Alternatively or additionally at least 30, 40, 50, 60, 70, 80 or 90% of product is obtained, as compared to the amount of product (e.g. one or more of alcohol, acid or ester) which is achieved following exposure of the same substrate to the protein encoded by the *almA* gene for the same time under the same conditions. Alternatively, the reduction in pour point is at least 30, 40, 50, 60, 70, 80 or 90% of that which is achieved following exposure of the same substrate to the protein encoded by the *almA* gene for the same time under the same conditions.

In a further alternative, the activity of the encoded enzyme can be described as being capable of complementing a mutation in the *almA* gene of *Acinetobacter* sp. DSM17874, i.e. capable of complementing a mutation leading to the inactivation of the gene encoded by or corresponding to SEQ ID NO:1 in *Acinetobacter* sp. DSMZ17874. It has been shown by the present inventors that this strain requires the product of the *almA* gene to be able to grow when the sole provided carbon source is a C32 *n*-alkane. A suitable assay for determining whether a particular strain is viable or can grow on a C32 *n*-alkane is also set out elsewhere herein, although any suitable method of determining cell viability or growth may alternatively be used. Proteins which share the catalytic function of the enzyme that is encoded by *almA* will be expected to be able to complement a mutation in *almA* in the *Acinetobacter* sp. DSM17874 strain which would otherwise fail to grow on a long-chain *n*-alkane substrate such as C32 *n*-alkane.

Whether or not a protein that is thought to share the catalytic activity of AlmA has this ability can readily be tested by expressing a gene that encodes the protein in a mutant strain of a microorganism which does not contain a functional *almA* gene and which cannot grow on a C32 *n*-alkane substrate (e.g. by using an *Acinetobacter* sp. DSM17874 strain in which the *almA* gene has been disrupted, deleted or otherwise altered so as to prevent the expression of a functional AlmA protein). Since the gene sequence for this protein is now known, this is straightforward to do (see e.g. Throne-Holst M et al., 2007 Appl Environ Microbiol. 73(10):3327-32).

The gene that encodes a protein that is thought to share the catalytic activity of AlmA can be transfected or transduced into the mutant strain using standard techniques that are known in the art. It will be necessary to place it under the control of an appropriate promoter and/or other regulatory sequences so that it is expressed in the mutant microorganism.

After a certain period of time, it can then be determined whether the exogenous gene has complemented the mutation in the mutant strain, i.e. whether its expression has rendered the mutant strain viable for growth on the C32 n-alkane substrate, and compensated for the absence of functional Alma. This can be achieved by testing for evidence of bacterial growth on the C32 n-alkane substrate. Where the exogenous expression of the protein allows the mutant strains to survive, i.e. it can compensate for the absence of expression of functional AlmA, the exogenous protein is said to have complemented the mutation. Genes whose expression can complement a mutation in the *almA* gene e.g. in the *Acinetobacter* sp. DSM17874 strain (e.g. strain MAV1 as described in the Examples) are considered to share the same activity as the AlmA protein.

The invention also extends to functional fragments of the above nucleic acid molecules, by which it is meant fragments that encode a protein which has the same or substantially the same activity (e.g. catalytic or enzymatic activity) as the full length protein as defined above. Tests to determine whether a protein encoded by such a fragment has the same or substantially the same activity (e.g. catalytic or enzymatic activity) as the full length protein as defined above include those discussed above. Normally these functional fragments will only have small deletions of nucleic acid molecules relative to the full length nucleic acid molecule, e.g. deletions of less than 50, 40, 30, 20 or 10 nucleotides, for example at the 5' end encoding the N-terminus of the protein, the 3' end encoding the C-terminus of the protein or internally within the encoding region, although larger deletions e.g. of at least 60, 70, 80, 90, 100, 150, 200, 300, 400, 500, 600, 700, 800, 900 or 1000 nucleotides, or deletions of less than 60, 70, 80, 90, 100, 150, 200, 300, 400, 500, 600, 700, 800, 900 or 1000 nucleotides can also be carried out, if the fragment has the same or substantially the same activity (e.g. catalytic or enzymatic activity) as the full length protein as defined above.

The activity of the encoded protein can readily be tested to determine whether it shares the same activity as the full length protein, e.g. as set out above.

Shorter fragments of the nucleic acid molecule of the invention can be used as probes, e.g. for PCR or hybridisation protocols. Shorter fragments can be e.g. 10-30, 20-25 nucleotides in length. Such probes are useful in protocols for identifying further nucleic acid molecules which share homology with the nucleic acid molecules of the invention.

The term "nucleic acid molecule" as used herein refers to a polymer of RNA or DNA that is single or double stranded, optionally including synthetic, non-natural or altered nucleotide bases. Examples of such polynucleotides include cDNA, genomic DNA and dsRNA, *inter alia.* Preferably, the nucleic acid molecule is DNA.

Whilst the nucleic acid sequences referred to herein comprise thymidine ("t") nucleotides, it will be understood that the invention also relates to corresponding sequences wherein thymidine is replaced by uridine ("u").

By degradation of long-chain *n*-alkanes it is meant the enzymatic use of a long-chain *n*-alkane, as described herein, as a carbon source. Alternatively defined, it is meant the enzymatic oxidation of a long-chain *n*-alkane, or the enzymatic conversion of a long-chain *n*-alkane into a molecule which has a shorter *n*-alkane chain length. Degradation of alkanes may lead to the production of esters, aldehydes, alcohols and/or acids.

Degradation may thus comprise or consist of one or more oxidation steps or reactions of the *n*-alkane, e.g. to convert the *n*-alkane to an alcohol, aldehyde, acid or ester. The conversion may result in an alcohol, aldehyde, acid or ester having the same chain length as the *n*-alkane before degradation or it may result in the production of a molecule having fewer carbon atoms than the *n-*alkane before degradation.

By n-alkane it is meant a straight chained alkane. As noted above, long-chain alkanes degraded by a putative monoxygenase of the invention are those having 26 or more carbon atoms, or 32 or more carbon atoms (e.g. 26-36, 28-34, or 30-32 carbon molecules). In contrast, medium-chain *n*-alkanes are *n*-alkanes having 11 to 20 carbon atoms, and short-chain alkanes have 1-10 carbon atoms. The long-chain *n*-alkanes are also sometimes called paraffins.

As indicated above, the invention also includes nucleic acid molecules comprising a variant of the nucleic acid molecule defined by SEQ. ID. No:1. The term "variant" includes nucleic acid molecules which have single or multiple nucleotide changes compared to the nucleic acid molecules of the invention. For example, the variants might have 1, 2, 3, 4, or 5 additions, substitutions, insertions or deletions of one or more nucleotides.

The invention also relates to nucleic acid molecules whose sequences are degenerate to the sequence of SEQ. ID. No: 1, i.e. molecules whose sequences contain nucleotide changes that do not result in a change in the encoded amino acid sequence. In general, however, such variants and degenerates satisfy the sequence criteria that are set out above.

In a further aspect, the invention provides a protein which is a putative monoxygenase and has a role in the degradation of an n-alkane substrate having 26 or more carbons comprising a sequence of amino acids selected from the group consisting of
(i) an amino acid sequence as set forth in SEQ ID NO:2, or
(ii) an amino acid sequence having at least 85% sequence identity, preferably at least 90, 95, 98% and more preferably at least 99% sequence identity, with an amino acid sequence as set forth in SEQ ID NO:2.

The protein may alternatively be defined with reference to the encoding nucleic acid sequences and as such the protein of the invention can be encoded by any of the nucleic acid molecules of the invention, as described above.

The invention also extends to functional fragments of the above protein molecules, by which it is meant fragments which have the same or substantially the same activity as the full length proteins as defined above i.e. they should be considered to be functionally equivalent variants. As noted elsewhere herein, the property can be tested for in various ways in a straightforward manner. Normally these functional fragments will only have small deletions relative to the full length protein molecule, e.g. of less than 50, 40, 30, 20 or 10 amino acids, although as noted above in connection with nucleic acid molecules larger deletions e.g. of up to 60, 70, 80, 90, 100, 150, 200 amino acids or at least 60, 70, 80, 90, 100, 150, 200 amino acids, may be appropriate. In all cases, the fragments should have the same or substantially the same activity as the full length proteins as defined above i.e. they should be considered to be functionally equivalent variants. These deletions may be at the N terminus, the C terminus or they may be internal deletions.

The protein of the invention as defined above includes variants of the specific, recited sequence, i.e. sequences having certain levels of sequence identity to the recited sequences. Such variants could be naturally occurring variants, such as comparable proteins or homologues found in other species or more particularly variants found within other microorganisms, (which share the functional properties of the encoded protein as defined elsewhere herein).

Variants of the naturally occurring protein as defined herein can also be generated synthetically e.g. by using standard molecular biology techniques that are known in the art, for example standard mutagenesis techniques such as site directed or random mutagenesis (e.g. using gene shuffling or error prone PCR). Such mutagenesis techniques can be used to develop enzymes which have improved or different catalytic properties.

A method for identifying residues in a long-chain *n*-alkane degrading protein as defined herein which are important for enabling long-chain *n*-alkane degradation, may comprise the steps of:
(i) mutating or otherwise altering one or more amino acid residues in the long-chain *n*-alkane degrading protein, and
(ii) testing the effect of the mutations or alterations on the property of long-chain *n-*alkane degradation.

In such a method, step (ii) can be performed by assessing the ability of the mutated or otherwise altered long-chain *n*-alkane degrading protein to cause degradation of a long-chain *n*-alkane substrate, e.g. by using the methods and assays as described elsewhere herein. A comparison of the ability of the mutated or otherwise altered long-chain *n*-alkane degrading protein to cause long-chain *n*-alkane degradation can then be made relative to the non- mutated or otherwise altered long-chain *n*-alkane degrading protein. The properties of the mutated or otherwise altered long-chain *n-*alkane degrading protein can thus be compared directly to the wild type, or parent sequence from which the mutated or otherwise altered long-chain *n*-alkane degrading protein is derived, e.g. the production of the various products, consumption of substrates and ability to modify the pour point of an *n*-alkane containing substrate.

In such methods, the mutations may have no effect on the degradation properties or may improve or reduce or inhibit the effect on degradation. The residues which when mutated have a positive or negative effect on the degradation properties are then identified as being important for function. Depending on the proposed use for the long-chain *n*-alkane degrading protein, such residues are either targets for further mutation (see below) or are identified as residues which should be retained in an unmodified form in order to preserve the important functional property of degradation of long-chain *n*-alkanes.

Alternatively viewed therefore, this provides a method for generating a long-chain *n*-alkane degrading protein with improved or increased ability to cause the degradation of a long-chain *n*-alkane,

It will be appreciated that a method of identifying, developing or producing other long-chain *n*-alkane degrading proteins, or variants thereof which have the ability to act on other substrates/polymers, may thus comprise the steps of:
(i) obtaining or otherwise providing a long-chain *n*-alkane degrading protein as defined herein,
(ii) mutating or otherwise altering one or more amino acid residues in the long-chain *n*-alkane degrading protein,
(iii) assessing the ability of said mutated or altered long-chain *n*-alkane degrading protein to degrade or to enhance the degradation of the substrate of interest,
(iv) identifying mutated or altered long-chain *n*-alkane degrading proteins which display the desired properties.

Appropriate substrates for use in the above methods could be any substrate of interest, in particular any long-chain *n*-alkane.

The long-chain *n*-alkane degrading protein used in these methods has the properties as defined elsewhere herein and may be a newly identified long-chain *n*-alkane degrading protein or may be a known molecule whose properties of degradation of long-chain *n*-alkanes have only become known by use of methods and assays as described herein.

Derivatives of proteins as defined above may also be used. By derivative it is meant a protein as described above or a variant thereof which instead of the naturally occurring amino acid, contains a structural analogue of that amino acid. Derivatisation or modification (e.g. labelling, glycosylation, methylation of the amino acids in the protein) may also occur as long as the function of the protein is not adversely affected.

By "structural analogue", it is meant a non-standard amino acid. Examples of such non-standard or structural analogue amino acids which may be used are D amino acids, amide isosteres (such as N-methyl amide, retro-inverse amide, thioamide, thioester, phosphonate, ketomethylene, hydroxyrnethylene, fluorovinyl, (E)-vinyl, methyleneamino, methylenethio or alkane), L-N methylamino acids, D-α methylamino acids, D-N-methylamino acids.

Sequence identity may be assessed by any convenient method. However, for determining the degree of sequence identity between sequences, computer programs that make multiple alignments of sequences are useful, for instance Clustal W (Thompson, J. D et al., 1994, "CLUSTAL W: Improving the sensitivity of progressive multiple sequence alignment through sequence weighting, position-specific gap penalties and weight matrix choice". Nucleic Acids Res 22: 4673-4680). Programs that compare and align pairs of sequences, like ALIGN (E. Myers and W. Miller, 1988, "Optical Alignments in Linear Space", CABIOS 4: 11-17), FASTA (W.R. Pearson and D.J. Lipman, 1988, "Improved tools for biological sequence analysis", PNAS 85:2444-2448, and W.R. Pearson, 1990, "Rapid and sensitive sequence comparison with FASTP and FASTA"Methods in Enzymology 183:63-98) and gapped BLAST ,(Altschul, S.F., et al., 1997, "Gapped BLAST and PSI-BLAST: a new generation of protein database search programs". Nucleic Acids Res. 25: 3389-3402) are also useful for this purpose. Furthermore, the Dali server at the European Bioinformatics institute offers structure-based alignments of protein sequences (Holm, 1993, J. of mol. Biology, 233: 123-38; Holm, 1995, Trends in Biochemical Sciences, 20: 478-480; Holm, 1998, Nucleic Acid Research, 26: 316-9). Multiple sequence alignments and percent identity calculations are preferably determined using the standard BLAST parameters, (using sequences from all organisms available, matrix Blosum 62, gap costs: existence 11, extension 1).

A further embodiment of the invention provides a chimeric gene comprising the isolated nucleic acid molecule of the invention operably linked to one or more suitable regulatory sequences. Preferably, the one or more of the regulatory sequences are heterologous regulatory sequences, for example, a heterologous promoter.

In the context of this invention, the term "operably linked" refers to the association of two or more nucleic acid molecules on a single nucleic acid fragment so that the function of one is affected by the other. For example, a promoter is operably linked with a coding sequence when it is capable of affecting the expression of that coding sequence (i.e. the coding sequence is under the transcriptional control of the promoter). Coding sequences may be operably linked to regulatory sequences in sense or antisense orientation.

The term "regulatory sequences" refers to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, operators, enhancers, translation leader sequences, introns, and polyadenylation recognition sequences.

As used herein, the term "promoter" refers to a nucleotide sequence capable of controlling the expression of a coding sequence or RNA. In general, a coding sequence is located 3' to a promoter sequence. Promoters may be derived in their entirety from a native gene, or be composed of different elements derived from different promoters found in nature, or even comprise synthetic nucleotide segments. It is understood by those skilled in the art that different promoters may direct the expression of a gene in different tissues or cell types, or at different stages of development, or in response to different environmental conditions. Promoters that cause a nucleic acid fragment to be expressed in most cell types at most times are commonly referred to as "constitutive promoters". It is further recognized that since in most cases the exact boundaries of regulatory sequences have not been completely defined, nucleic acid fragments of different lengths may have identical promoter activity.

A further embodiment of the invention provides a vector comprising the nucleic acid molecule of the invention operably linked to one or more suitable regulatory sequences.

Vectors comprising the instant isolated nucleic acid molecule of the invention (or chimeric gene of the invention) may be constructed. The choice of vector is dependent upon the method that will be used to transform host cells, the method that is used for protein expression, or on another intended use of the vector. The skilled person is well aware of the genetic elements that must be present on the vector in order to successfully transform, select and propagate host cells containing the nucleic acid molecule or chimeric gene of the invention. The skilled person will also recognize that different independent transformation events will result in different levels and patterns of expression and thus that multiple events must be screened in order to obtain lines displaying the desired expression level and pattern. Such screening may be accomplished by Southern analysis of DNA, Northern analysis of mRNA expression, Western analysis of protein expression, *inter alia.*

The invention further provides microorganisms that contain one or more of the nucleic acid molecules of the invention, and hence are capable of expressing one or more of the proteins of the invention. Such microorganisms may have been genetically manipulated to contain the appropriate nucleic acid molecule of the invention or a genetic construct comprising the appropriate nucleic acid molecule or chimeric gene of the invention, e.g. in the form of the above described vectors of the invention.

An example of a microorganism containing the nucleic acid molecules of the invention, and which naturally expresses these nucleic acid molecules is the *Acinetobacter* sp. DSM17874 strain (deposited as DSM 17874 on 31 January 2006 in the Deutsche Sammlung von Mikroorganismen und Zellkulturen GmbH (German Collection of Microorganisms and Cell Cultures)). The strain has also been deposited at the DSMZ under the conditions of the Budapest Treaty on 20 June 2007, by Statoil ASA, under accession number DSM19446. The present invention thus relates to the *Acinetobacter* sp. DSM17874 strain (deposited as DSM 17874 and as DSM19446)

Microorganisms may be genetically manipulated so that they contain one or more of the nucleic acid molecules of the invention and/or so that they express the protein encoded by one or more of the nucleic acid molecules of the invention. In the former case, the encoding sequence i.e. one or more of the nucleic acid molecules of the invention is artificially introduced into the microorganism. This can be in the form of one or more of the chimeric genes of the invention, or one or more of the vectors of the invention. Such microorganisms can be described as exogenously containing one or more of the nucleic acid molecules of the invention.

In a further alternative, the microorganism endogenously contains one or more of the nucleic acid molecules of the invention, but the microorganism is genetically manipulated so as to alter the expression of the products encoded by the one or more nucleic acid molecules of the invention. This can be achieved for example by introducing a further copy of the nucleic acid molecule of the invention under the control of a different promoter, e.g. a strong promoter or a constitutive promoter, or by genetic manipulation of the promoter which controls the expression of the nucleic acid of the invention. In both of the above cases, genetic material is present in the microorganism that is not present in the naturally-occurring equivalent microorganism (i.e. exogenous genetic material is present).

In a preferred embodiment therefore, the microorganism contains exogenous genetic material, i.e. the microorganism is genetically modified.

In general, the exogenous genetic material is introduced using the process of transformation. Transformation will typically involve a plasmid vector which will also contain a gene to enable identification of successfully transformed microorganisms, e.g. a gene for antibiotic resistance (for example against ampicillin). Other methods for selecting transformants are known to the skilled person and include the use of a light sensitive vector, a lux-gene, which causes positive colonies to light up in the dark. Other suitable vehicles for transformation of the bacteria include cosmids and bacteriophage molecules.

The microorganism is preferably a bacterium or an Archeaum. Microorganisms may be extremophilic, e.g. thermophilic. Suitable microorganisms may also be halophilic and anaerobic. Suitable examples include sulphur reducing bacteria like *Desulfovibrio-, Desulfobulbus-, Desulfobacter, Desulfococcus-* strains etc., different *Bacillus* species, *Clostridium* species, *Thermoanaerobacter, Thermoanaerobium, Thermobacteroides, Thermodesulfobacterium,* some *Pseudomonas* etc. Some sulphur reducing bacteria may also use other metabolic pathways through lactate or acetate.

Special considerations are needed for cloning genes into *Thermus* species. To clone a heterologous gene into *Thermus* bacteria, the respective gene must be either (1) cloned into an insertion vector between sequences of *Thermus* origin, which following transformation, will lead to insertion of the gene into the chromosome by homologous recombination or (2) the gene has to be inserted into a vector containing an origin of replication for *Thermus.* Shuttle vectors, containing an origin of replication for both *E. coli* and *Thermus,* are often used, which allow construction of plasmid variants in *E. coli* with subsequent transformation of *Thermus* with the resulting plasmid variants. As such, conventional methods used for cloning of genes in *E. coli* are applied for establishing *Thermus* plasmid constructs, either for insertion or replication. Subsequently, the *Thermus* bacteria are transformed with plasmid DNA isolated from *E. coli.* Two established *Thermus* transformation methods are described below.

The first uses their natural transformation system and is based on the method of Koyama et al., 1986, J. Bacteriol. 166:388-340 and Fridjonsson et al., 1999 Appl. Environ. Microbiol. 67:3955-3964).

A fresh overnight Thermus culture is diluted 1:100 in T162 medium (Degryse, E., et al., 1978, Arch. Microbiol. 117:189-196). The culture is grown at 65°C for 3 - 4 hours in the presence of 2 mM MgCl₂ and CaCl₂ respectively, or until the culture has reached about 0.8 at OD₆₀₀. 500 µl of the culture is then dispensed into an eppendorf tube.

The transformation DNA is then added into the culture and shaken at 60°C for 1- 2 hours. The amount of DNA depends on the source and the form of the DNA. For example about 200ng of DNA of *Thermus* origin can be used, or 1- 3 µg of DNA from *E.coli* (cloned DNA) can alternatively be used).

The culture is then cooled on ice for approximately 5 minutes and plated on T162 medium using appropriate selection agent(s) and grown at 60° for 48 hours.

An alternative methodology is based on electroporation (e.g. of *Thermus* HB8 and HB27 (de Grado et al., (1999) Plasmid. 42:241-5).

Cells are grown exponentially up to about 0.8 at OD₆₀₀ and spun down at 5000 g for 5 min. The cells are then washed 2 x in a 10% glycerol solution at room temperature, and concentrated 10 x after the second wash. This results in the cell density reaching up to 2 x 10¹⁰ (HB8) and 5 x 10¹⁰ (HB27) respectively.

100 µl cell aliquots are incubated at 4 °C for 5 min with 1 pg of DNA. The cells are electroporated using a current strength of 12,5 kV/cm (maximum) during a 5 ms pulse. These parameters are identical are identical to the conditions Biorad's Micropulser delivers using the preset program "Ec2" using a 0.2 cm cuvette (see manual for Biorad MicroPulser (Cat. No 165 - 2100) pp 4 for specifics).

Cells are allowed to recover for 2 hours at 65 - 70°C under aeration before plating on T162 medium using appropriate selection agent(s).

In addition to the above-described properties of the microorganism, it is possible to further manipulate the microorganism to add or remove enzymatic or other activities from the microorganism. As noted above, a further gene encoding e.g. an antibiotic resistance protein may be inserted so as to assist with the process of selecting genetic transformants. Alternatively or additionally, nucleic acid molecules which are designed to influence the degradation of n-alkanes or other compounds can also be introduced into the microorganism.

As is well known, the introduction of exogenous nucleic acid molecules into a microorganism may lead to the microorganism having an activity which is not found in the unmodified microorganism (e.g. where the microorganism is supplemented with an additional activity, such as a nucleic acid molecule encoding an enzyme that is not found in the unmodified microorganism) or else the exogenous nucleic acid molecule that is to be introduced may be designed so that it interferes with the normal transcription and/or translation of one or more genes whose product would be otherwise expressed in the microorganism (i.e. the expression of one or more genes in the modified microorganism is reduced or compromised or eliminated). The latter can be achieved by various means such as gene disruption following homologous recombination or the use of antisense or siRNA.

In a preferred example, the microorganism expresses one or more of the proteins of the invention (either endogenously or through the introduction of exogenous nucleic acids as described above), and in addition, does not express genes which encode enzymes involved in the degradation of short-chain *n*-alkanes or does not express genes which encode enzymes involved in the degradation of medium-chain *n-*alkanes or *n*-alkanes with a chain length of C20-C24.

For example, the microorganism may contain no endogenous genes which encode enzymes that are involved in the degradation of short-chain *n*-alkanes or does not contain endogenous genes which encode enzymes involved in the degradation of medium-chain *n*-alkanes or *n*-alkanes with a chain length of C20- C24. Such a microorganism could be transformed with an appropriate nucleic acid molecule such that it expresses one or more of the proteins of the invention.

Alternatively, the microorganism may contain endogenous genes which encode enzymes that are involved in the degradation of short-chain *n*-alkanes and/or genes which encode enzymes involved in the degradation of medium-chain *n*-alkanes or *n-*alkanes with a chain length of C20- C24 but the expression of one or more of these endogenous genes may be interfered with, e.g. using the techniques mentioned above. In addition the microorganism should express one or more of the proteins of the invention (either endogenously or through the introduction of exogenous nucleic acid).

In all cases, the enzymes involved in the degradation of medium-chain alkanes or short-chain alkanes can be any enzyme which catalyses at least one step in this process (e.g. monooxygenases, rubredoxins and rubredoxin reductases, alcohol dehydrogenases, aldehyde dehydrogenases, alkane hydroxylases and acyl coA synthetases/fatty acid-CoA ligases).

Examples of genes which encode enzymes involved in the degradation of n-alkanes with a chain length of C10-C18 are the *AlkMa* and/or *AlkMb* genes (having GenBank accession numbers DQ009004 and DQ009005, respectively). A preferred microorganism is thus a mutant of the *Acinetobacter* sp. DSM17874 strain (deposited as DSM 17874) in which the expression of the *AlkMa* and/or *AlkMb* genes has been disrupted or interfered with, e.g. a knock-out or deletion mutant of the *AlkMa* and/or *AlkMb* gene has been made. Further, preferred embodiments are strains which do not contain *AlkMa* and/or *AlkMb* genes endogenously, or functional equivalents thereof, but which do contain the nucleic acid molecule of the invention (either endogenously or through the introduction of exogenous nucleic acids as described above).

The protein of the invention or the microorganisms of the invention (or a cell extract thereof) as described above may be formulated with a carrier liquid. This may be the form in which the protein of the invention or the microorganisms of the invention is applied to the *n*-alkane containing substrate. The invention thus provides a composition comprising these components.

Viewed from another aspect, the invention provides the use of the protein of the invention or the microorganisms of the invention (or a cell extract thereof) as described above for the manufacture of treatment compositions for degrading long-chain *n*-alkanes, lowering the pour point of a long-chain *n*-alkane containing substrate, or causing esterification of long-chain *n*-alkanes. In all cases the long-chain *n*-alkane referred to is at least 26 or 32 carbons in length.

Various polymeric, oligomeric, inorganic and other particulate carriers to which proteins and/or microorganisms can be attached are known, e.g. ion exchange resin particles (see US-A-4787455), acrylamide polymer particles (see EP-A-193369), gelatin capsules (see US-3676363), oligomeric matrices and capsules (see US-A-4986353 and US-A-4986354), ceramic particles (see WO 99/54592, WO 96/27070 and EP-A-656459), and particles of the well treatment chemical itself (see WO 97/45625.

Viewed from a further aspect the invention thus provides particles impregnated with the protein of the invention or with the microorganisms of the invention as described above.

The protein of the invention or microorganisms of the invention as described above can also be attached to a filter (e.g. for down-hole placement).

The invention provides a method of degrading long-chain *n*-alkanes in a long-chain alkane containing substrate, preferably crude oil, said method comprising exposing said long-chain *n*-alkane containing substrate to a protein that is capable of degrading long-chain *n*-alkanes (e.g. the protein of the invention). As discussed above, this process of degradation can have several consequences, e.g. it can lead to the production of compounds containing a shorter *n*-alkane chain, e.g. further *n-*alkanes, or to the production of esters, alcohols, aldehydes and/or acids. Alternatively or additionally the pour point of the substrate may be reduced.

By long-chain *n*-alkane containing substrate it is meant any substrate which contains one or more long-chain *n*-alkanes as defined above. Preferably the substrate is crude oil. It can alternatively comprise or consist of purified or partially purified long-chain *n*-alkanes, HC-fractions (e.g. hydrocarbon fractions that are obtained from crude oil by fractional distillation) and plant or animal fat and oils.

It is thus clear that there are various different advantages to modifying the various substrates according to the invention. Reduction of the viscosity of the substrate in the context of oil production and refinery will assist in production and movement of the oil. In addition, causing degradation of the substrate as described above can alter the composition of the substrate, e.g. such that it contains a higher proportion of alcohols, acids, esters or aldehydes. These various components can be separated according to standard methods and utilised for various chemical syntheses. Alternatively, it is also known that oil containing oxidised components can be more valuable. By choosing an appropriate substrate and controlling the exposure of the substrate to the *n*-alkane degrading proteins, the properties of the substrate can hence be manipulated as desired.

By "exposing" the substrate containing long-chain *n*-alkanes to a protein that is capable of degrading long-chain *n*-alkanes (e.g. the protein of the invention), it is meant that the substrate and protein are brought into contact in an appropriate context and under appropriate conditions so as to allow the protein to interact with the long-chain *n*-alkanes component of the substrate so that the protein may carry out its activity on i.e. the long-chain *n*-alkanes.

The long-chain *n*-alkane containing substrate is thus mixed with, or contacted with an appropriate protein or protein source under suitable conditions so as to allow this interaction to take place. Suitable contact must therefore be made between the protein and the long-chain *n*-alkane so as to allow these two components to interact. Thus, the protein may simply be brought into contact with the long-chain *n*-alkane substrate for example by adding it directly to the long-chain *n*-alkane substrate. This contact may be achieved in a bioreactor, which may be a batch reactor or a continuous reactor. Alternatively, the contact may be achieved during the industrial oil production process, e.g. in a pipe, pipeline or well.

It should be noted that any appropriate conditions can be used during this process, and the step of "exposing" the long-chain *n*-alkane substrate to the protein of the invention can take place in any appropriate context, e.g. in a bioreactor or stirring tank or in the context of the industrial oil production process (e.g. in a pipeline, pipe or well) in which case the step of exposure could take place down hole. For example suitable proteins, lysates or whole microorganisms, immobilised or in free form could be injected into the near well bore area. In all cases, immobilized proteins, lysates or organisms can be used, as can free live organisms, as discussed in detail elsewhere herein.

It will be appreciated by the person skilled in the art that the necessary incubation times, pH, temperature, substrate concentrations and protein concentrations are not independent of each other. Thus, a large range of conditions can be envisaged, which can easily be evaluated by a person skilled in the art.

In the method of the invention the protein, microorganism or cell extract thereof may be placed *in situ* before and/or after the substrate. Preferably the protein, microorganism or cell extract thereof is placed *in situ* before the substrate, especially in the case of construction of pipes or other vessels.

The above-described step of exposure of the substrate to an appropriate protein or protein source may be achieved by contacting the substrate directly with the protein, or else by contacting the substrate with a microorganism which is capable of expressing the protein. The protein can be exposed to the substrate alone, or in combination with one or more other proteins. Examples of other proteins that can be used include monooxygenases, rubredoxins and rubredoxin reductases, alcohol dehydrogenases, aldehyde dehydrogenases, alkane hydroxylases and acyl coA synthetases/fatty acid-CoA ligases. In a further alternative, an extract of a microorganism which is capable of expressing the protein can be used. Suitable microorganisms include those which express an appropriate protein (e.g. the protein of the invention) and these are discussed above. Any of the microorganisms discussed above could be used.

Appropriate forms of the protein of the invention and variants etc., thereof, which can be used in the methods of the present invention, are described above. Thus, the protein of the invention that is present can be in many different forms, e.g. isolated, extracted or purified from various different sources or synthesised by various different means.

For example, the protein that is used (e.g. the protein of the invention (or variant, etc., thereof)) can be a synthetic protein that has been chemically synthesised. Chemical syntheses may be performed by methods well-known in the art involving, in the case of peptides, cyclic sets of reactions of selective deprotection of the functional groups of a terminal amino acid and coupling of selectively protected amino acid resides, followed finally by complete deprotection of all functional groups. The proteins of the invention for use in the invention may be substantially purified, e.g. pyrogen-free, e.g. more than 70%, especially preferably more than 90% pure (as assessed for example, in the case of peptides or proteins, by an appropriate technique such as peptide mapping, sequencing or chromatography). Purification may be performed for example by chromatography (e.g. HPLC, size-exclusion, ion-exchange, affinity, hydrophobic interaction, reverse-phase) or capillary electrophoresis.

Recombinant expression of proteins is also well known in the art and an appropriate nucleic acid sequence can be used to express an appropriate protein (e.g. the protein of the invention (or variant, etc., thereof)) for subsequent expression and optional purification using techniques that are well known in the art. For example, an appropriate nucleic acid sequence can be operably linked to a promoter for expression of the protein in bacterial cells, e.g. *E coli.* It may be convenient to express the protein under the control of an inducible promoter, in which case the expression of the protein will be induced in culture to cause expression.

As an alternative to carrying out purification, lysates or extracts of the cell expressing the nucleic acid molecule of the invention may serve as an alternative source of the protein (e.g. the protein of the invention). Cell extracts or lysates can be made according to standard techniques that are known in the art, e.g. by sonication, or other means of cell disruption, by using detergents. The lysate and/or purified protein can then be mixed with an appropriate liquid or solution for administration or addition to the substrate that is to be exposed thereto. This approach is equally applicable to recombinant bacteria and to bacteria that naturally synthesise the protein (e.g. the protein of the invention).

Partial purification or concentration of the appropriate protein from the lysate of the bacteria which naturally produces the protein or which is expressing the protein recombinantly is also possible. Suitable examples of how this might be carried out to achieve the required concentration or purity are well known in the art. Alternatively, a microorganism which expresses an appropriate protein can be contacted with the long-chain n-alkane containing substrate. This approach is equally applicable to recombinant bacteria and to bacteria that naturally synthesise such a protein (e.g. the protein of the invention).

The microorganism, lysate or extract thereof or protein may be delivered to the site where it is to act on the substrate alone or in or on particles, e.g. porous inorganic particles (e.g. Silica, alumina etc.) or polymeric particles or in capsules, e.g. colloids, etc. These carrier particles may also carry bacterial nutrients. Such particles are discussed above and are well known in the art.

As noted above, the step of exposing the substrate to an appropriate protein can occur in a bioreactor, or pipes or pipelines, well or other vessels or elsewhere in the industrial oil process.

The method steps that are described above can alternatively be used to achieve a reduction in the pour point of a long-chain *n*-alkane containing substrate such as crude oil.

These method steps can also alternatively be used to achieve an increase in the flow of a long-chain *n*-alkane containing substrate such as crude oil, e.g. in pipelines and/or the conversion of long-chain *n*-alkanes, into alcohols, acids and/or esters.

The above references to reduction in the context of the methods mean that following the method steps, the appropriate parameter has been reduced by any amount, e.g. a statistically significant amount. For example in connection with a reduction in pour point, it is meant that the pour point is lowered e.g. by at least 0.1, 0.25, 0.5, 1, 1.5, 2, 3, 4, 5, 6, 7, 8, 9, 10, 15, or 20 degrees (in °F or °C) relative to substrate that has not been exposed to the protein or microorganism of the invention.

As discussed above, the inventors were able to identify the above-described gene through the development of a high throughput screening method, which enabled them to identify genes that are involved in the degradation of long-chain *n*-alkane substrates. The method uses mutant strains which are tested for their ability to grow on substrates in two or more selection steps, and the combination of these two steps allows the identification of mutations that specifically affect the ability of the mutation present in each mutant strain to affect growth on one particular substrate. The substrates are chosen such that the unmutated or parent strain can grow on each of the substrates that are used in the selection procedure (e.g. a long-chain *n*-alkane such as a C32 *n*-alkane and a medium-chain n-alkane such as a C16 *n*-alkane). The gene of interest is identified by identifying mutant strains that do not grow on one substrate (e.g. a long-chain *n*-alkane such as a C32 *n*-alkane) but are viable on the other substrate (e.g. medium-chain *n*-alkane such as a C16 *n*-alkane). Mutant strains that have these properties thus contain a mutation that is specific to the pathway for degrading one of the substrates only. The combination of selection steps in this method thus eliminates the identification of mutants that contain mutations that affect the general viability of the cell, and which are not specific to the degradation of the substrate of interest.

Thus, there is additionally now taught a method of identifying a gene in a microorganism that encodes a protein that is required for the metabolism of a substrate comprising the steps of
a) providing a library of mutant strains of bacteria which have been obtained by mutagenesis of a parent microorganism strain,
b) subjecting the mutant strains to a set of growth conditions for negative selection,
c) identifying clones that are growth deficient or nonviable in this set of growth conditions,
d) subjecting the mutant strains to a set of growth conditions for positive selection,
e) identifying clones that can grow or are viable in this set of growth conditions, and
f) identifying the gene that is mutated in the identified clones
wherein the parent microorganism grows or is viable when subjected to set of growth conditions for negative selection and grows or is viable when subjected to the set of growth conditions for positive selection and wherein the set of growth conditions for negative selection is such that the substrate of interest is present and the set of growth conditions for positive selection is such that the substrate of interest is absent and a different substrate is present.

The above method may be used to identify genes in any microorganism. Preferably the microorganism is a bacterium or Archaeaum.

Microorganisms can grow on various substrates or growth media in an experimental setting. In many cases it is possible to grow microorganisms on a very simple mixture of nutriments and salts. Whilst in a natural setting any particular microorganism may be growing by metabolising or degrading multiple carbon and energy sources which are present in the area in which is grows, it is possible to learn more about the particular metabolic requirements of microorganisms by growing microorganism on basic or minimal growth medium and supplementing the basic growth medium with a specific substrate or a well defined set of substrates. The ability of microorganisms to grow on these substrates is then determined. For example, the basic growth medium may be supplemented with a particular *n*-alkane as a liquid or solid, alone or in combination with other *n*-alkanes or other substrates.

There will often be a range of substrates on which the microorganism can grow and the ability of the microorganism to grow on any one substrate indicates that the microorganism has one or more genes which encode proteins that can degrade that substrate. Similarly, the inability of a mutant strain of the microorganism to grow on such a substrate may indicate that the mutant strain has a mutation in a gene which affects the ability of the strain to degrade the substrate on which it is grown, or else it may have a mutation in a gene which otherwise affects the viability of the strain. As discussed above, the method of the inventors distinguishes between mutations in genes which are specific to the degradation of a particular substrate, and genes which otherwise affect cell growth and/or viability.

Furthermore, since the metabolic pathways in a microorganism are not all distinct, linear pathways, there are proteins in a microorganism which are needed for the microorganism to be able to metabolise a variety of related or distinct substrates, (such as in the *alk* pathway where there are several copies of the initial alkane hydroxylating enzyme, AlkM). These variants of the same enzyme have slightly different substrate specificities. After the initial step, the subsequent degradation follows the same pathway. The method described above allows for this by the presence of the counterselection step (positive selection). The two substrates used for the negative and positive selection steps can be chosen so as to identify a protein which lies in the metabolic pathway of the substrate of interest at an appropriate position. For example, if it is required to identify a protein that is required for the metabolism of a C32 *n*-alkane substrate, then a parent strain which grows on this substrate and also on a distinct substrate such as a short-chain (e.g. C6) *n*-alkane, or a medium-chain *n*-alkane (e.g. C16) substrate, is selected and mutant strains are tested for their ability to grow on these two substrates. Such a method would identify genes encoding proteins that are essential for the degradation of C32 *n-*alkanes but not for the degradation of C16 *n*-alkanes, and was used to identify the genes of the invention, and whilst it is possible that such a method might identify genes whose products are required for the metabolism of C32, 36, 20 and 24 *n-*alkanes, the *almA* gene product at least does not appear to be required for growth on C20 and C24 alkanes although it is required for growth on C32 and C36 alkanes (see Example 2).

If it were required to identify a protein that is required specifically for the metabolism of a C32 *n*-alkane substrate, but not for other long-chain alkanes such as C20 and C24 alkanes, it would be possible to perform the counterselection step on a substrate such as C20 and/or C24, or else to perform one or more additional counterselection step on additional substrates such as C20 and/or C24. Indeed, in Example 2 the initial selection step was based on the absence of growth of mutant clones on C32 but growth on C16. Further characterisation of the growth abilities of the mutants (by growing them on a selection of different substrates) can also be carried out to narrow down the selection procedure, and the exposure to these different growth conditions could be carried out in parallel or sequentially.

In view of the above considerations it can be seen that by "required for the metabolism of' it is meant any molecule, such as a protein (in particular an enzyme) that is needed for a microorganism to grow on the specified substrate, but that is not needed for the general viability of the cell. Preferably, the method is used to identify molecules, e.g. proteins that are required for the specific metabolism of a certain substrate, in that they are not required for the metabolism of other, distinct substrates. Preferably therefore the method is used to identify molecules, e.g. genes encoding proteins that are required for growth on long-chain *n*-alkanes (or at least one long-chain *n*-alkane) but not required for growth on medium or short-chain n-alkanes. More preferably, the method is used to identify genes encoding proteins that are required for growth on long-chain *n*-alkanes (or at least one long-chain n-alkane) having at least 26, 28, 30, 32 or 34 Carbons but not required for growth on shorter-chain n-alkanes such as C24 or C16.

It is clear from the above discussion that the skilled person would be able to identify and select an appropriate combination of a microorganism and substrates, based on the information provided above so as to be able to carry out the method in order to identify a gene having particular property.

By library it is meant a collection or set of individuals. The mutant strains that are members of the library are all derived from the same parent strain in that they are produced by mutagenesis of the same parent strain. They thus share characteristics and features with the parent strain, but differ in that they contain mutations in their genome.

The mutations may be generated by any convenient means, such as exposure of the parent strain to chemical mutagens, or insertional mutagenesis such as the use of transposon mutagenesis, as set out in Example 1. Preferably the mutagenesis is carried out such that each member of the library contains a single mutation.

As discussed above, the growth conditions are selected such that in the first set of growth conditions the substrate of interest is present. The substrate may be added to the growth medium in any suitable form. For example, solid *n*-alkanes may be added as a powder to the growth medium on the surface of solid growth medium or as a droplet into liquid growth medium, and liquid *n*-alkanes may be added as a liquid drop either to solid or liquid growth medium.

The growth medium can be any standard basic or minimal medium which allows growth of the microorganism, when supplemented with an appropriate carbon source in the form of the added substrate of interest. Other appropriate supplementary compounds may also be added, where necessary (e.g. antibiotics for selection purposes).

Examples of suitable substrates include long-chain n-alkanes, e.g. one or more of C20, C22, C24, C26, C28, C30, C32, C34, C36, C38, C40, C42, C44 *n*-alkanes. As discussed above, appropriate substrates are chosen depending on the nature of the gene that is intended to be identified.

The substrate of interest is added to the growth medium at an appropriate concentration to enable growth to be sustained for sufficient time for a colony to be formed, e.g. for at least 2-3 days, preferably at least 3-5 days.

In general the clones are plated out for this culture step, e.g. in 96-well plates or omnitray plates such that the location of each clone within the plate is known. The retention of replica plates will be useful to allow for replating of identified clones. Any suitable culture apparatus may be used which allows the subsequent identification of the clones to be carried out.

In general, the strains are incubated for 2 to 3 days under appropriate culture conditions (e.g. approximately 30°C or 37°C, 70-75°C or 65°, depending on the nature of the microorganism).

This step may be performed in parallel for more than one substrates or carbon sources, depending on the nature of the gene that is intended to be identified, for example it would be possible to grow the clones on both C16 and C32 simultaneously, and then identify those that were able to grow on C16 but not on C32 for subjection to further analysis.

As discussed above, the growth conditions are selected such that in the positive set of growth conditions the substrate of interest is absent, and an alternative carbon source or substrate is present. As is the case for the first substrate (i.e. the substrate of interest) the alternative substrate may be added to the growth medium in any suitable form.

Also as discussed above, the growth medium can be any standard minimal medium which allows growth of the microorganism, when supplemented with an appropriate carbon source in the form of the added substrate of interest. The growth medium for this set of growth conditions may be the same (apart from the supplementary substrate) or different from the growth medium for the negative set of growth conditions, but preferably it is the same, i.e. it has the same composition. Other appropriate supplementary compounds may also be added, where necessary (e.g. antibiotics for selection purposes).

Examples of suitable substrates for inclusion in the second set of growth conditions include short-chain *n*-alkanes, medium-chain *n*-alkanes, or long-chain alkanes with fewer than 30 carbon atoms e.g. one or more of C6, C8, C10, C12, C14, C16, C18, C20, C22, C24, C26, C28 *n*-alkanes. A further alternative is to use acetate, since this will enable the exclusion of any mutants carrying mutations in "house-keeping" genes. As discussed above, appropriate substrates can be chosen depending on the nature of the gene that is to be identified, e.g. genes encoding proteins involved in metabolic pathways common to all microorganisms.

The substrate of interest is added to the growth medium at an appropriate concentration to enable growth to be sustained for at least 2-3 days, preferably at least 3-5 days.

In general, the clones are plated out for this culture step, e.g. in 96 well plates or omnitray plates such that the location of each clone within the plate is known. Replica plates can be retained to allow for replating.

In general the strains are incubated for 2 to 3 days under appropriate culture conditions (e.g. approximately 30°C or 37°C, 70-75°C or 65°, depending on the nature of the microorganism).

As discussed above, the identified clones may be subjected to further selection steps, based on the nature of the gene that it is intended to identify. For example, where a gene involved in the metabolism of C32 n-alkanes is sought, the first set of growth conditions can be growth on C32, the counterselection or second step of growth conditions can be growth on C16, and those that do not grow on C32 but do grow on C16 can then be subjected to growth on additional n-alkane substrates such as C20 or C24. Such additional steps can be carried out for example if the number of clones that are identified in step (e) is large. A further alternative is to use acetate, since this will enable the exclusion of any mutants carrying mutations in "house-keeping" genes, e.g. genes encoding proteins involved in metabolic pathways common to all microorganisms.

In steps (c) and (e) referred to above, a distinction is made between "growth deficient" clones and those that are viable. In each of these steps it is necessary to identify those clones which can grow under the particular growth conditions to which they have been subjected. By this it is meant that clones that are unable to grow or to survive on a particular substrate are distinguished from those which are able to grow.

Viable cells, which grow in the particular growth conditions used can be identified by observing a measurable or detectable amount of cell growth e.g. there may be a statistically significant increase in cell number, for example at least a fivefold, tenfold, twenty-fold, thirty-fold or fifty-fold increase in cell number, for example as measured by appropriate means e.g. spectrophotometrically by determining an increase in absorbance of the cell culture (e.g. growth medium) or increase in optical density e.g. OD₆₀₀ or other spectrophotometric or colorimetric assays for proteins or other cell components e.g. by staining the colonies with a compound which can detect viable clones, such as INT (iodonitrotetrazolium chloride) or by measuring the amount of protein per liter of culture. This growth may be detected in any convenient or desired way.

A clone is deemed to be growth deficient if there is no increase in cell number following the incubation under the specified growth conditions, if there is only a limited increase in cell number following the incubation under the specified growth conditions and/or if there is no detectable living cellular material following the incubation under the specified growth conditions. Non-viable clones are detected through the absence of detectable living cellular material following incubation under the specified growth conditions. By limited increase it is intended to refer to a small number of cell divisions, e.g. representative of 2-5 cell divisions.

The process of identifying growing and non-growing, and viable and non-viable clones may be facilitated by the use of appropriate controls. For example, in parallel with the test plates in each culture step the clones may be replicated into a control plate and incubated under conditions which are known to sustain the growth of the parental strain, preferably under conditions under which the growth of any mutant strains would not be anticipated to be influenced by mutations to the gene of interest, as a positive control. All strains should grow under these conditions. A growth medium which contains a mixture of different carbon sources, such as the widely available LB broth would be an appropriate growth medium to use in such a positive control. A further positive control is to use the parental strain and to measure or observe the growth of that strain in the various test culture conditions. A negative sample or plate may be grown under conditions which are known not to sustain the growth of the parental strain, e.g. the basic or minimal growth medium without any additional source of carbon.

Comparisons between the amount of growth seen in these positive and negative controls, with the clones will assist in determining whether any particular clone should be considered to be growth deficient or viable under any particular test conditions.

The clones can be exposed to the growth conditions in any order, i.e. the culturing step and the associated step of identifying clones that are growth deficient in the set of culture conditions for negative selection may take place before, after or simultaneously with the culturing step and the step of identifying clones that are viable in the set of culture conditions for positive selection.

The process is, however, facilitated if the steps are performed sequentially since each identification step will eliminate the need to investigate particular clones further. As such, a preferred gene identification method comprises the steps of
a) providing a library of mutant strains of bacteria which have been obtained by mutagenesis of a parent microorganism strain,
b) subjecting the mutant strains to a set of growth conditions for negative selection,
c) identifying clones that are growth deficient or nonviable in this set of growth conditions,
d) subjecting the clones identified in step (c) to a set of growth conditions for positive selection,
e) identifying clones that can grow or are viable in this set of growth conditions, and
f) identifying the gene that is mutated in the identified clones
wherein the parent microorganism can grow or is viable when subjected to the set of growth conditions for negative selection and can grow or is viable when subjected to the set of growth conditions for positive selection and wherein the set of growth conditions for negative selection is such that the substrate of interest is present and the set of growth conditions for positive selection is such that the substrate of interest is absent and a different substrate is present.

A further preferred gene identification method comprises the steps of
a) providing a library of mutant strains of bacteria which have been obtained by mutagenesis of a parent microorganism strain,
b) subjecting the mutant strains to a set of growth conditions for positive selection,
c) identifying clones that can grow or are viable in this set of growth conditions,
d) subjecting the clones identified in step (c) to a set of growth conditions for negative selection,
e) identifying clones that are growth deficient or nonviable in this set of growth conditions,
f) identifying the gene that is mutated in the identified clones
wherein the parent microorganism can grow or is viable when subjected to the set of growth conditions for negative selection and can grow or is viable when subjected to the set of growth conditions for positive selection and wherein the set of growth conditions for negative selection is such that the substrate of interest is present and the set of growth conditions for positive selection is such that the substrate of interest is absent and a different substrate is present.

Further selection steps may be added as appropriate, as discussed above.

Once mutant clones have been identified which fulfill the growth requirements of the method, the gene which has been disrupted or mutated in the clone is identified. This may be carried out using standard molecule biology techniques known in the art, e.g. PCR based methods and/or Southern blotting. If the library of mutants has been generated by transposon mutagenesis, it will be possible to use known sequences from the transposon sequence to assist in cloning the mutated region. The method thus optionally includes further steps of cloning and sequencing the mutated gene.

The invention will now be described in more detail in the following non-limiting Examples in which
Figure 1 shows a physical map of pLOFKm. The transposase gene and kanamycin resistance marker are indicated; the mini-Tn10 is flanked by two IS10 insertion sequences.
Figure 2 shows a picture of an omnitray plate filled with CB solid medium and C32 applied as a powder on top of it. Cells were replicated from a 96-well plate onto the plate and grown overnight at 30 °C. Approximately 20 ml INT top agar were applied subsequently to detect viable cells. Some colony spots are missing due to an abandoned growth of the respective clones under the chosen conditions (indicating that there is no growth of these colonies on C32).
Figure 3 shows physical maps of pSOK804 (A) and pLAL50 (B). Ori: pBR322 replication origin, Am^{R}: apramycin resistance marker, oriT: origin of transfer. Restriction sites (*XbaI* and *BamHI*) and cloned fragments are indicated.
Figure 4 shows growth curves of strains *Acinetobacter* sp. DSM17874 (wild-type), AVM1004A7 (transposon mutant strain) and MAV1 (*αlmA* knockout strain) grown in CB medium with antibiotics and C20, C24, C32, and C36 as sole carbon sources.
Figure 5 shows a schematic drawing of the surrounding regions of the homologous genes *almA* in *Acinetobacter venetianus 6A2* (*Acinetobacter* sp. DSM17874) and ACIAD3192 in *Acinetobacter* sp. ADP 1 designated as putative monooxygenase, flavin-binding family. *rpsM, rpsK, rpoA,* and *rplQ* represent ribosomal proteins, *fadE* encodes an acyl-CoA dehydrogense. *almB* represents a potentially secreted protein of yet unknown function. An involvement of Orfl (AlmB) in alkane metabolism possibly linked to AlmA is currently under investigation. Unlabelled elements are speculative.
Figure 6 shows the nucleotide sequence of the *almA* coding region
Figure 7 shows the peptide sequence of the almA protein
Figure 8 shows the nucleotide sequence of the *orf1* (*almB*) coding region
Figure 9 shows the peptide sequence of the Orf1 (AlmB) protein
Figure 10 shows the *almA-orf1 (almAB)* locus.

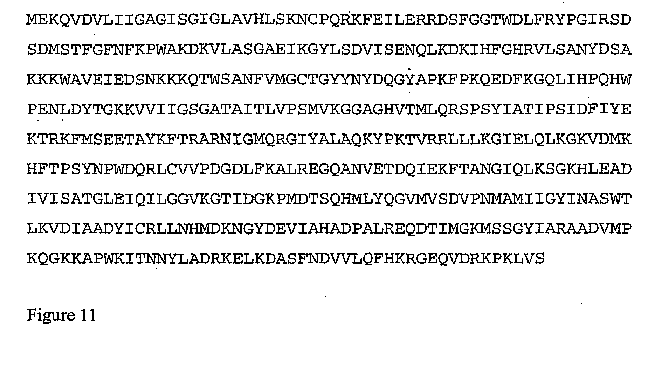

Figure 11 shows the peptide sequence of *Acinetobacter* sp. M-1 AlmA

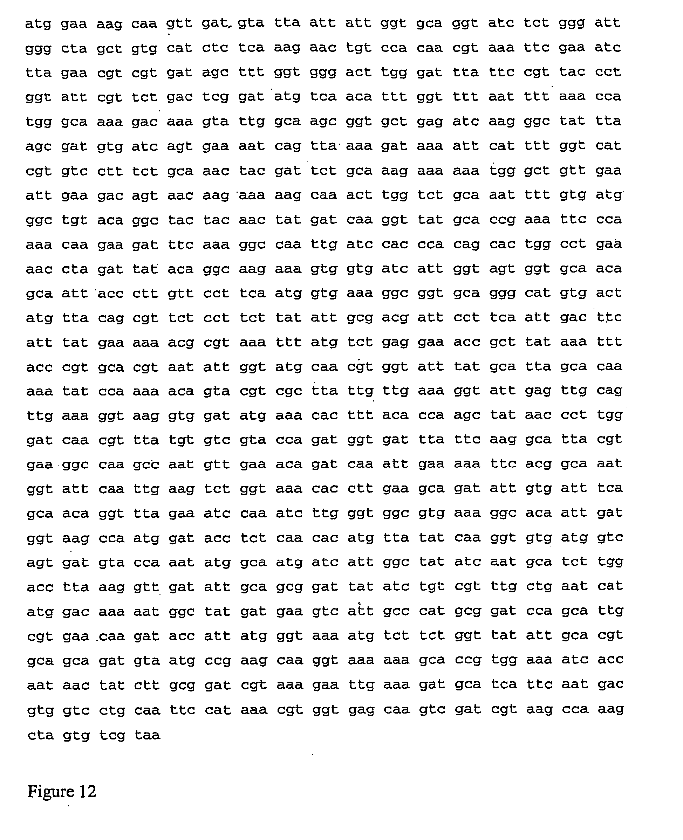

Figure 12 shows the nucleotide sequence of *Acinetobacter* sp. M-1 AlmA
Figure 13 shows the results of complementation of *almA* activity in the *almA-*deficient mutant *Acinetobacter* sp. MAV1 with the *almA* gene on plasmid. Growth of *Acinetobacter* sp. DSM 17874/pDLM02.1 (o), *Acinetobacter* sp. MAV1/pDLM02.1 (□), *Acinetobacter* sp. MAV1/pALMA1 (∇) and *Acinetobacter* sp. MAV1/pACIAD1 (Δ) in 100 mL CB medium supplemented with seven 50-µL solid droplets of C32 (A) or C36 (B). Growth was measured as an increase in protein in the cultures over time. The values presented are the averages of two independent experiments.

### EXAMPLES

### Example 1 Generation of the Acinetobacter venetianus 6A2 (Acinetobacter sp. DSM17874) transposon library

The *Acinetobacter* sp. DSM17874 strain transposon library was constructed by conjugational transfer of pLOFKm from *E. coli* S17-1 (λ pir) harboring the plasmid to *Acinetobacter* sp. DSM17874 strain and selection for chromosomal integration of the transposon.

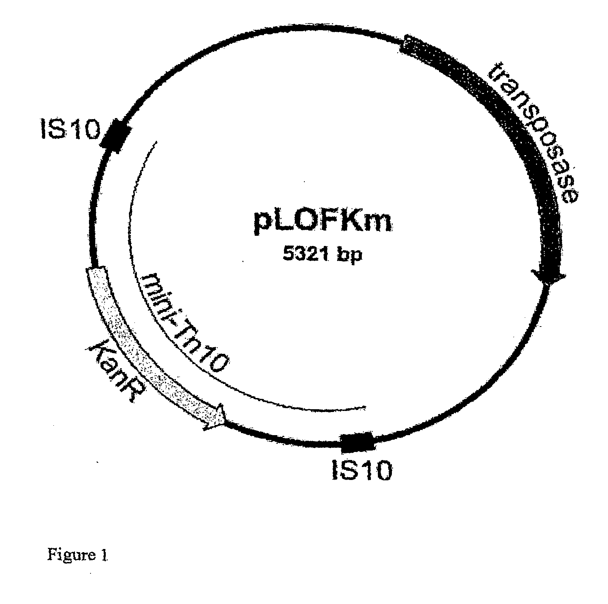

*Acinetobacter* sp. DSM17874 was re-streaked from -80 °C 25 % glycerol culture on LB plates containing 25 µg/ml chloramphenicol. *E. coli* S17-1 (λ pir) harboring plasmid pLOFKm (Figure 1) was re-streaked from -80 °C 25 % glycerol culture on LB plates containing 150 µg/ml ampicillin and 75 µg/ml kanamycin. Both plates were incubated overnight at 30 °C. 50 ml LB medium (per liter: 10 g tryptone, 5 g yeast extract, 10 g NaCl; for plates: additional 15 g/L agar bacterial.) containing the mentioned concentrations of antibiotics were inoculated with material from the plates and incubated overnight at 30 °C).

The next day 50 ml LB medium containing the mentioned concentrations of antibiotics were inoculated with 500 µl from the two 50 ml pre-cultures. The *E. coli* culture was started 2 h after the 6A2 culture. After growth *of Acinetobacter* sp. DSM17874 for 4.5 h and E. coli S17-1(λ pir)/pLOFKm for 2.5 h cultures showed an O.D.₆₀₀ of approx. 0.4. 1 ml of each of the two cultures were mixed and centrifuged at 8000 rpm, 5 min, RT. The pellet was re-suspended in 100 µl LB medium and placed as a droplet on top of an LB plate containing 500 µM IPTG. The plate was sealed with parafilm and incubated upside-down at 30 °C overnight.

The next day, cells were harvested from the LB/IPTG plate in 500 µl LB medium and plated in dilutions on LB plates containing 150 µg/ml ampicillin and 75 µg/ml kanamycin (10⁵, 10⁶, 10⁷), containing 25 µg/ml chloramphenicol (10⁵, 10⁶, 10⁷), and on transposon selective plates. For the latter 43x 100 µl of the 10x dilution was plated onto 43 large LB plates containing 25 µg/ml chloramphenicol, 75 µg/ml kanamycin and 500 µM IPTG. All plates were incubated overnight at 30 °C. The next day the donor to recipient ratio was determined to be 1:587 and the total number of isolated clones was counted to be approx. 6800. These clones from the transposon selective plates were picked and transferred to 71x 96-well plates (AVM1A-AVM71A) containing 150 µl LB medium per well containing 75 µg/ml kanamycin using a Qpix robot. These 71 plates form the AVM library. The plates were incubated overnight at 30 °C shaking at 500 rpm in an incubator at 85 % humidity.

The plates were replicated into new 96-well plates containing 150 µl LB medium with 75 µg/ml kanamycin per well. These so called B-series plates (AVM1B-AVM71B) were again incubated at 30 °C shaking at 500 rpm in an incubator at 85 % humidity overnight. After replication of the initial plates to form the B-series and replication for the C32 screening experiment (see below), the initial plates and the plates of the B-series were frozen at -80 °C after addition of 50 µl 50 % glycerol per well and sealing of the plates.

### Example 2 High throughput screen for strains deficient for growth on C32

For the high-throughput screening experiment the initial library plates AVM1A to AVM71A were replicated onto omnitray plates with CB solid medium (per liter: 3 g NaNO₃, 1 g K₂HPO₄, 0.5 g MgSO₄, 0.5 g KCI, 0.01 g FeSO₄; for plates: additional 15 g/L agar bacterial) containing 75 µg/ml kanamycin onto which C32 alkane was added as a powder using a sterile tea sieve. The solid alkane was prepared by placing sterile *n*-alkane droplets into a sterile mortar, adding liquid nitrogen and thoroughly grinding the droplets). The plates were incubated at 30 °C for two (AVM36A-AVM71A) and three days (AVM1A-AVM35A) respectively.

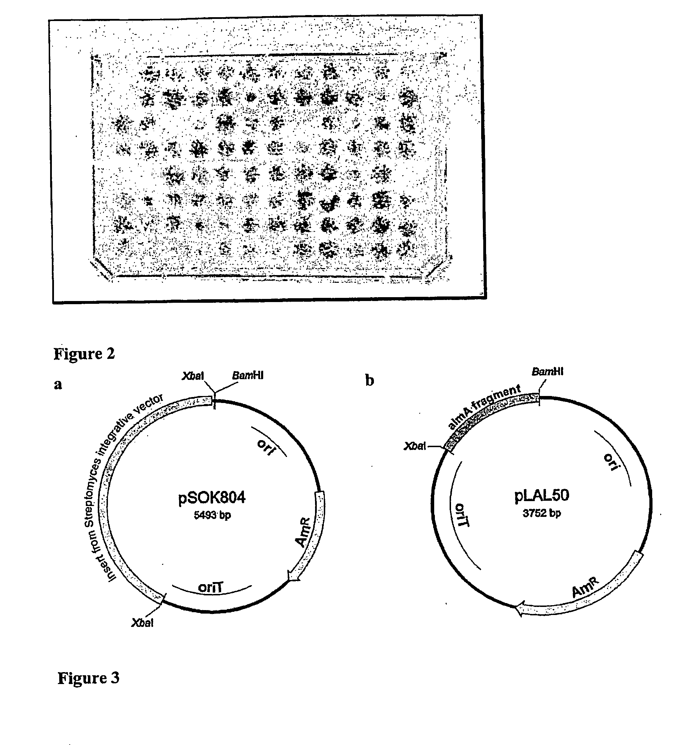

To identify clones in the AVM library not able to grow on C32 the replicated omnitray plates containing CB solid medium (75 µg/ml kanamycin, C32 alkane added) were stained by adding approx. 20 ml INT top agar (CB medium, 0.05% INT, 1.5 % agar) to each omnitray plate. After a few minutes 'colonies' became visible by showing a red color (for an example see Figure 2).

Mutants which showed no or poor growth with C32 alkane as sole carbon source (475 clones) were re-arrayed into a total of 6x 96-well plates filled with 120 µl LB medium containing 75 µg/ml kanamycin per well using the Qpix robot (Genetix). The re-array was done using the B-series plates AVM1B-AVM71B which were thawed and re-frozen during the re-arraying. The re-arrayed plates (AVM1001-AVM1004 for clones with no growth and AVM1005-AVM1006 for clones with poor growth) were incubated overnight at 30 °C shaking at 500 rpm in an incubator at 85 % humidity.

Clones from the re-arrayed plates AVM1001-AVM1006 were replicated onto the following omnitray plates:
- LB + 75 µg/ml kanamycin
- LB + 75 ug/ml kanamycin + 25 µg/ml chloramphenicol
- CB + 75 µg/ml kanamycin + 0.5 % sodium acetate
- CB + 75 µg/ml kanamycin + C16 alkane (400 µl C16 alkane was added to the plate lids, droplets were formed by autoclaving the solid n-alkanes and pipetting of the liquid in 50 µl aliquots onto a sterile petri dish)
- CB + 75 µg/ml kanamycin + C32 alkane (C32 alkane was added as a powder)

The plates were incubated overnight at 30 °C. This replica plating and counter-selection is necessary for identification of clones with impaired or abandoned growth only on the C32 alkane. The identification of genes involved in long-chain alkane degradation is only possible from mutant clones showing impaired growth solely on long-chain alkanes and exhibiting normal growth on shorter-chain alkanes and on complex medium. This procedure excludes mutant clones with transposon interruptions in genes not involved in C32 alkane degradation.

From the replicated plates, 20 clones showed no growth on C16 and C32 alkanes and normal growth on LB medium and on CB medium containing acetate. 34 clones did not grow with C32 alkane as the sole carbon source but showed growth on C16 alkane, LB, and CB + acetate. These latter 34 clones were characterized further with respect to growth on various alkanes.

1 µl (using 1 µl inoculation loops) of each of the 34 mutants were re-suspended in 200 µl LB medium. The cell suspensions were used for plating of the strains on CB solid medium containing 75 µg/ml kanamycin and supplemented with one of the following carbon sources: 0.5 % acetate, C12, C16, C20, C22, C24, C32, C36 alkanes. Liquid alkanes were added as 400 µl to the lids, solid alkanes were added as powders. The plates were incubated at 30 °C. The growth of the clones was recorded and 12 mutant strains still growing on C24 alkane and at the same time not growing on C32 and C36 alkanes were chosen for further analysis. 12x 3 ml LB medium containing 25 µg/ml chloramphenicol and 75 µg/ml kanamycin were inoculated with the 12 relevant mutant strains (AVM1001H3, E6, A11; AVM1002C8, AVM1003E4, F5, G6; AVM1004A2, C4, D5, A6, A7). The cultures were incubated overnight at 30 °C and 200 rpm on a shaking incubator for strain conservation and preparation of chromosomal DNA. After incubation, the clones were frozen at -80 °C) in a final concentration of 25 % glycerol.

### Example 3 Identification of almA

From 2 ml of each of 11 cultures mentioned above chromosomal DNA was prepared using the DNAeasy Tissue Kit (Qiagen) according to the protocol for Gram-negative bacteria. The chromosomal DNA was digested with *Hind*III and subsequently religated after thermal inactivation of the endonuclease.

PCR on the ligates using oligonucleotides Kan1(5'GCTCTAGACCGTCAAGTCAG CGTAATGC-3'SEQ ID NO:6) and Tn10:1 (5'-GGATCATATGACAAGATGTG-3' SEQ ID NO:7) as primers yielded PCR products for AVM1001H3, AVM1001E6, AVM1003E4, AVM1003F5, AVM1003G6, AVM1004A2 (2 products), AVM1004A6, AVM1004C4, AVM1004D5, and AVM1004A7). The PCR products were isolated after agarose gel electrophoresis using the Qiaquick Gel Extraction Kit (Qiagen). Direct sequencing was performed on the purified PCR products using oligonucleotides Tn10:1 (AVMS1, 3-10, 15-18) and Kan1 (AVMS2; only AVM1001H3) as primers in cycle sequence. AVMS3 and 7 (AVM1001E6 and AVM1004A2) did not yield readable sequences and were re-done as AVMS 15 and AVMS16, 17 on a new batch of PCR product. AVMS1, 6, 8, and 9 (AVM1001H3, AVM1003G6, AVM1004C4, AVM1004D5) turned out to be identical. The obtained sequences were used for BlastN database searches.

The clone AVM1004A7 harboring the transposon disruption in a putative monooxygenase gene (later named *almA)* potentially involved in *Acinetobacter* sp. DSM17874's long-chain alkane degradation was investigated with respect to growth on long-chain alkanes in comparison with the constructed knockout mutant strain MAV1.

Based on the initial sequencing results of AVM1004A7 oligonucleotides could be defined for amplification of chromosomal DNA around the transposon insertion site. On a PCR product obtained with oligonucleotides AVM1004A7-1 (5'-ACCCTGTACAACCCATTACG-3' SEQ ID NO:8) and AVM1004A7-2 (5'-AGTTGTGATCATCGGCAGTG-3' SEQ ID NO:9), sequencing was performed using AVM1004A7-1 and AVM1004A7-2 (AVMS13-14) as sequencing primers. The results expanded the known *almA* sequence information to about 600 bp, sufficient for construction of a knockout mutant strain (MAV1) using a suicide vector.

### Example 4 Construction of the almA knockout mutant strain MAV1

To generate an *almA* knockout mutant strain a sufficiently long *almA* DNA sequence must be cloned into a vector not able to replicate in *Acinetobacter* sp. DSM17874 and transferable from *E. coli* S17-1(λ pir) to *Acinetobacter* sp. DSM17874 via conjugation. A vector matching these requirements is pSOK804 (Sekurova et al., 2004, J Bacteriol. 186(5): 1345-54, Figure 5A). Using chromosomal DNA of *Acinetobacter* sp. DSM17874 as a template, a PCR was performed using oligonucleotides AVM1004A7-3 5'-GCTCTAGACTATCCTGGTATTCGTTCAG-3' SEQ ID NO:10) and AVM1004A7-4 5'-CGGGATCCTAAATACCACGTTGC ATACC-3' SEQ ID NO:11) as primers. The PCR product was isolated from an agarose gel using the Qiaquick Gel Extraction Kit (Qiagen). The PCR product and the vector pSOK804 were digested with *BamHI* and *XbaI.* Both vector fragment and digested PCR product were isolated by gel electrophoresis and subsequent gel extraction using the Qiaquick Gel Extraction Kit (Qiagen). Vector and PCR product were ligated to give plasmid pLAL50 (Figure 3B). *E. coli* S17-1 (λ pir) competent cells were transformed with the ligation product. Plasmid DNA was prepared from transformants, and cloning of the *almA* fragment was confirmed by restriction digest analysis. Conjugation was performed between *Acinetobacter* sp. DSM17874 and *E. coli* S17 1 (λ pir)/pLAL50 similar to the procedure described above for generation of the transposon library. After selection for resistance against apramycin (provided by pLAL50 integration) and chloramphenicol (*Acinetobacter* sp. DSM17874 is naturally resistant to chloramphenicol), two conjugants could be detected. The MAV1 clones were verified by Southern blot analysis according to the Roche manual using an *almA* specific probe.

### Example 5 Characterization of the almA mutant strains AVM1004A7 and MAV1

From a 25 % glycerol containing -80 °C storage culture cells of *Acinetobacter* sp. DSM17874 (1), AVM1004A7 (2) and MAV1 (3) were streaked on LB plates containing 30 µg/ml chloramphenicol (1), 50 µg/ml kanamycin (2) or 50 µg/ml apramycin (3). The plates were incubated overnight at 30 °C. A single colony of each strain was re-streaked on antibiotics-containing LB plates which were again incubated overnight at 30 °C. Two selective LB plates for each strain were subsequently inoculated with streaks from the re-streaked plates and used as pre-cultures after another overnight incubation to obtain enough biomass for inoculation. The cells were collected from the plates in each 6 ml CB medium and were used for inoculation after determination of the O.D.₆₀₀.

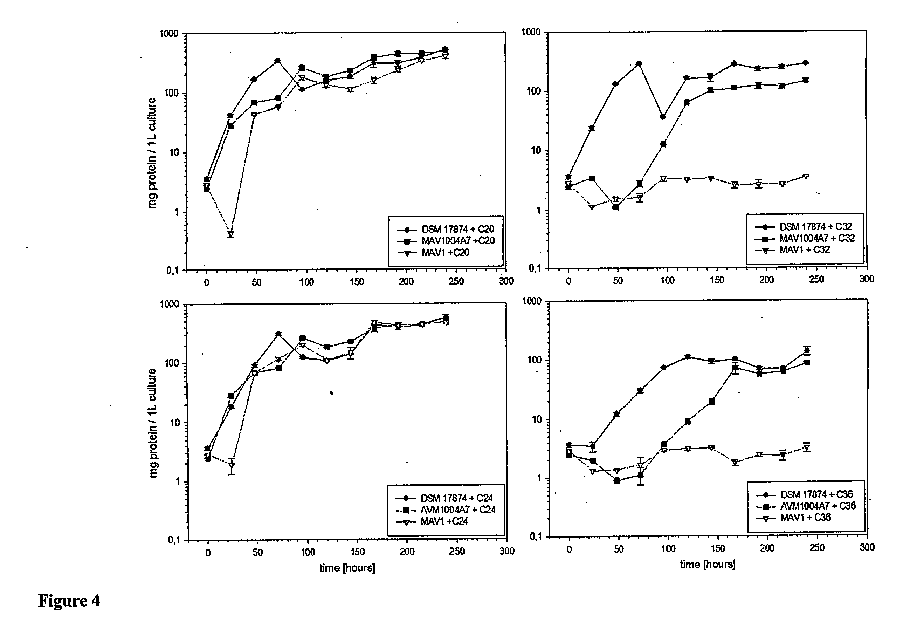

100 ml CB medium containing no antibiotics (1), 50 µg/ml kanamycin (2) or 50 µg/ml apramycin (3) were supplemented each with 7 x 50 µl solid droplets C20, C24, C32 and C36 alkanes. The medium was inoculated with bacterial cells of the three strains to a final O.D.600 of ~0.05 (1: 0.064, 2: 0.056, 3: 0.066). The cultures were grown at 30 °C with shaking at 200 rpm for 2 weeks. 2 ml culture samples were taken every 24 h, and bacterial cells from the samples were pelleted in a microcentrifuge (15000 rpm, 5 min, RT). The supernatant was disposed and the bacterial pellet was stored at -20 °C prior to protein content determination. After final sampling all samples of the series were thawed at RT, and the bacterial pellet was re-dispersed in 400 µl dH₂O (2 ml reaction vial). The sample was incubated in an ultrasound bath for 10 min and subsequently boiled in a water bath for another 10 min. 2 x 160 µl of the evenly dispersed sample were analyzed in a microtiter plate as duplicates using the Bio-Rad protein assay concentrate. Dilutions with dH₂O were used if required. Dilutions of BSA (80, 70, 50, 30, 10, 8 and 5 µg/ml in dH₂O) were used in duplicate as a standard on each plate. 40 µl of the concentrate were added to the diluted samples and the standards. The plate was incubated on a MT plate shaker at 900 rpm for 30 min prior to photometric measurement at 595 nm wavelength. The samples' protein concentrations were determined according to the BSA standard curve and the dilution factor. The protein concentrations were plotted against time to obtain growth curves using SigmaPlot 9 (Figure 4).

All three strains showed comparable growth on C20 and C24 alkanes starting immediately after inoculation. On C32 and C36 alkanes strain *Acinetobacter* sp. DSM17874 grew well (comparable to C20 and C24 alkanes) while AVM1004A7 first showed no growth and started growing after approx. 100 h of incubation. MAV1 first showed no growth up to about 250 h before growth of the culture could be detected.

These results show that presence of a functional *almA* gene is essential for growth of *Acinetobacter* sp. DSM17874 on long-chain alkanes such as C32 and C36, and suggests a central role of *almA* in utilization of these n-alkanes. The fact that both AVM1004A7 and MAV1 started growing within the time-frame of the growth experiment may be due to degradation of the antibiotics kanamycin and apramycin. Absence of the antibiotics would relieve selective pressure on maintenance of the integrated transposon or vector, and can allow reversion to wild-type genotype and phenotype with a functional *almA* through homologous recombination or transposon excision.

### Example 6 Complete sequence determination for the almA gene

Chromosomal DNA of AVM1004A7 was isolated and digested with *PvuI.* The digested fragments were re-ligated and used as a template in subsequent PCRs. A PCR using oligonucleotides Tn10:1 and Kan2 as primers yielded an approximately 4.1 kb PCR product. Direct sequencing was performed on this PCR product using oligonucleotides Tn10:1 and Kan2 as primers. The sequences (AW1, AW2) revealed DNA sequence upstream of the *almA* gene (30S ribosomal protein S11).

Three PCRs were performed to amplify the almA region in two fragments. A first PCR (1) using AVM1004A7-4 and AVMext2 (5'-CAAGGTAATGCATTGGC TTGGGCTACCTCAG-3' SEQ ID NO:12) as primers lead to a product of approximately 4.1 kb (5' part); the second PCR reaction (2) using AVM1004A7-2 and AVMext1 (5'-TTCTATGAAAAAGAAGTTGTTCCAAATATTG-3' SEQ ID NO:13) as primers resulted in a product of approximately 2.6 kb (3' part); a third PCR (3) using AVM1004A7-3 and AVMext1 as primers yielded a product of approximately 3.2 kb (3' part, elongated). Using PCR products (1) and (2) as templates, direct sequencing was performed using oligonucleotides AVM1004A7-1 (1: AW5) and AVM1004A7-2 (2: AW6). These sequencing results (AW5, AW6) allowed the definition of oligonucleotides for further sequencing (AW6) and revealed sequence information including the *almA* promoter region and the ATG start codon (AW5).

The PCR product (3) was cloned into plasmid pMOSBlue using the pMOSBlue Blunt-ended PCR Cloning Kit (Amersham). Sequencing of plasmid DNA prepared from three individual clones (M3: AW15-17; M5: AW19-21; M6: AW23-25) was performed using AVM1004A7-2 (AW15, AW19, AW23), AVM1004A7-3 (AW16, AW20, AW24) and AVM1004A7-5 (5'-CGTTTATGTGTTGTGCCAGATGGTG-3' SEQ ID NO:14, AW17, AW21, AW25). The sequences AW5 and AW15-17, AW19-21, and AW23-25 were composed to give one contig including the complete *almA* gene including the promoter region and sequence information for the region downstream of *almA.*

### Example 7- Complete sequence determination of the orf1 (almB) gene

From the sequences AW17, AW21, and AW25 after a 124 bp intergenic region the start codon for another open reading frame could be identified. To expand the sequence information on this putative gene, further sequencing was performed using the above mentioned plasmid clones M3 (AW27, AW30), M5 (AW28, AW31), and M6 (AW29, AW32) as a template and oligonucleotides AVMext3 (5'-GAAATATCGCGTATATTGAGCACATTAG-3' SEQ ID NO:15, AW27-29) and AVMext4 (5'-ATTGTCGATATATGCGCGTGC-3' SEQ ID NO:16, AW30-32) as primers in the sequencing reaction.

These two steps of 'primer walking' revealed the complete sequence of the new open reading frame henceforth named *almB,* and closed the gap to the 3-prime end of the next downstream gene *fadE* (acyl-CoA dehydrogenase) previously identified as a gene downstream of *almA* with opposite orientation. The relative positions of all genes identified are shown in Figure 7.

The sequences that were obtained above were then verified in the following manner. To amplify the *almAB* nucleotide sequence, four individual PCRs were performed using chromosomal DNA *of Acinetobacter* sp. DSM17874 as template and primers: almA-prom-up (GACATGTGTATTGTCAAATTTGTGC SEQ ID NO:17) and almB-end-lo/orfl-end-lo (CCAATGAGATCATGGAAGAAC SEQ ID NO:18). To minimize potential errors, the Expand High Fidelity Mix (Roche) was used containing a polymerase with proof-reading activity, the expected PCR product of 2272 bp was obtained in all of the 4 reactions. The product was run on a 0.8 % agarose gel and the four PCR products were excised subjected individually to gel extraction using the Qiaquick Gel Extraction Kit (Qiagen).

The PCR products were ligated in parallel into vector pMOSBlue from the pMOS Blue Blunt-ended PCR Cloning Kit (Amersham) followed by transformation according to the manufacturers manual. Six clones of each cloning showing white colonies were subjected to PCR screening using primers AVMext3 (GAAATATCGCGTATATTGAGCACATTAG SEQ ID NO:15) and almB-end-lo. Clones showing the expected PCR product were identified for clonings 1, 2, and 3.

From these clones, mini plasmid preparations were performed using the Wizard Plus Minipreps DNA Purification System (Promega). Restriction analysis of the plasmid DNA using *EcoR*I revealed clones AB1.1 (cloning 1, clone 1), AB2.6 (cloning 2, clone 6) and AB3.5 (cloning 3, clone 5) showing the expected bands in gel electrophoresis analysis. These three plasmid clones were used for sequence determination of the *almA*B region.

The sequencing was performed using the following primers in sequencing reactions AW109 to AW141.

| Primers | Clone 1.1 | Clone 2.6 | Clone 3.5 |
|---|---|---|---|
| ***forward*** | | | |
| *almA-prom-up* | AW109 | AW110 | AW111 |
| *AVM1004A7-2* SEQ ID NO:9 *(AGTTGTGATCATCGGCAGTG)* | AW112 | AW113 | AW114 |
| *AVM1004A7-5* SEQ ID NO*:14 (CGTTTATGTGTTGTGCCAGATGGTG)* | AW115 | AW116 | AW117 |
| *AVMext3* | AW118 | AW119 | AW120 |
| | AW121 | AW122 | AW123 |
| ***reverse*** | | | |
| *AVM1004A7-6* SEQ ID NO:20 | AW124 | AW125 | AW126 |
| *(GAGAGATGTACAGCTAACCCAATCCC)* | | | |
| *AVM1004A7-1* SEQ ID NO:8 *(ACCCTGTACAACCCATTACG)* | AW127 | AW128 | AW129 |
| *AVM1004A7-4* SEQ ID NO:11 *(CGGGATCCTAAATACCACGTTGCATACC)* | AW130 | AW131 | AW132 |
| | AW133 | AW134 | AW135 |
| | AW136 | AW137 | AW138 |
| *almB-end-lo* | AW139 | AW140 | AW141 |

The following sequence reactions could be assembled to give one contig of the *almAB (almA-orf1)* region: AW109-141 except for AW115, AW116, AW121, AW122, AW123, AW135, AW137, and AW138. The results are given in Figures 6-10.

The derived consensus sequence of the *almA* and *almB* coding regions (Figures 6 and 8), the putative AlmA and AlmB peptide sequences derived from the gene sequences (Figures 7 and 9) and the sequence of the complete *almAB (almA-orf1)* region (Figure 10) are shown, based on forward and reverse sequences of three independent clones.

The *almA* coding region consists of 1494 bp encoding a protein of 498 amino acids. The sequences for both the DNA coding region and the deduced peptide are given in Figures 6 and 7. The AlmA peptide sequence shows 76.0 % homology to a protein from the entirely sequenced strain *Acinetobacter* sp. ADP1 (*A baylyi* ADP1) in a simple BlastP search. The protein ACIAD3192 is predicted to be a putative flavin-containing monooxygenase with no specific functional assignment. The identity of the ADP 1 monooxygenase-encoding gene to the *almA* sequence on the nucleotides basis was determined to be 74.6 % using BlastN. Predictions for secondary structure (TMpred) suggested AlmA as a putative membrane protein of the inner bacterial membrane with three transmembrane α-helices.

*A. baylyi* ADP1 and *Acinetobacter* sp. RAG-1 and M-1, were also found to grow with C32 and C36, respectively, as a sole carbon source (results not shown and Sakai, Y et al., 1994, Biosci Biotechnol Biochem 58:2128-2130). Genes homologous to *almA* and *almB* were also identified in *Acinetobacter* sp. RAG-1 and *Acinetobacter* sp. M-1, using PCR with the primers AlmA prom-up (GACATGTGTATTGTCAAATTTGTGC SEQ ID NO:17) and almB end lo and almB-end-lo (CCAATGAGATCATGGAAGAAC SEQ ID NO:18) followed by primer walking. The sequence of the *Acinetobacter baylyi* ADP1 ACIAD3192 gene referred to above was obtained from GenBank, accession no. NC_005966 (2).

An *almB (orf1)* homolog is not present in *Acinetobacter* sp. ADP1. The genes of *almA* and *almB (orf1)* have the same orientation and may potentially form an operon. The *almB (orf1)* gene presumably encodes a 166 amino acid protein containing an N-terminal signal sequence for secretion of the predicted protein. The function of *almB (orf1)* is currently unknown. Database searches revealed no significant homologies to other known proteins. Nevertheless, the close association with *almA* may point to an association of *almB* with the ability of *Acinetobacter* sp. *DSM17874* to degrade long-chain *n*-alkanes.

### Example 8 complementation of almA activity in the almA-deficient mutant Acinetobacter sp. MAV1

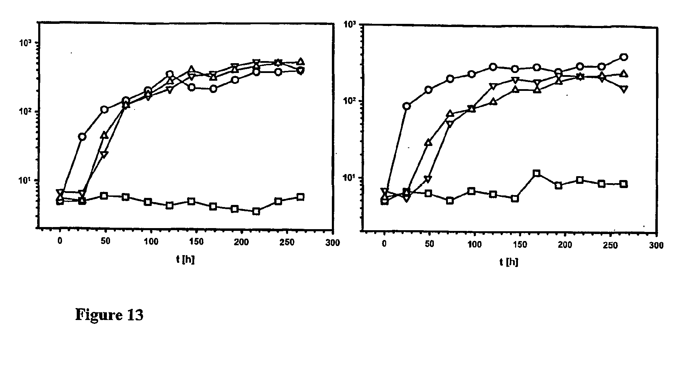

Introduction of the *almA* gene on a plasmid in the *almA*-deficient strain MAV1 restored the strain's ability to grow with C32 and C36 alkanes as a sole carbon source to almost wild-type level: (Fig. 13). The plasmid was made as follows, the *almA* gene of *Acinetobacter* sp. DSM 17874 was amplified by PCR using chromosomal DNA from *Acinetobacter* sp. DSM 17874 as a template. The PCR product was digested with *Sph*I and *Nar*I and ligated into similarly digested vector pDLM02.1 giving plasmid pALMA1.

This result confirmed that the long-chain alkane degradation deficiency of MAV1 is not caused by a polar effect on the genes downstream of *almA,* and that the *almA* deficiency is responsible for the inability of the MAV1 mutant to utilize long-chain alkanes.

The ACIAD3192 gene from *A. baylyi* ADP1 represents the closest homologue to the *almA* gene found in the databases. Therefore, this gene was chosen for heterologous functional complementation of the *almA*-deficient mutant MAV1. *Acinetobacter* sp. The plasmid was made as follows, the ACIAD3192 gene from *A. baylyi* ADP1 was amplified by PCR using chromosomal DNA from *A. baylyi* ADP1 as a template. The PCR product was digested with *Sph*I and *Nar*I and ligated into similarly digested vector pDLM02.1 giving plasmid pACIAD1.

MAV1 carrying the ACIAD3192 gene on a plasmid showed growth with C32 and C36 alkanes (Fig. 13), clearly showing that the product of this gene is a functional homologue of AlmA in the long chain alkane utilization pathway.

## Claims

1. A nucleic acid molecule which encodes a putative monoxygenase protein that has a role in the degradation of an n-alkane substrate having 26 or more carbons, comprising a nucleic acid molecule selected from the group consisting of:
(i) a nucleic acid molecule whose nucleotide sequence is set forth in SEQ ID NO: 1,
(ii) a nucleic acid molecule whose nucleotide sequence has at least 80% sequence identity with a nucleic acid molecule whose nucleotide sequence is set forth in SEQ ID NO: 1,
(iii) a nucleic acid molecule encoding the protein whose amino acid sequence is set forth in SEQ ID NO: 2,
(iv) a nucleic acid molecule whose nucleotide sequence has at least 85% sequence identity with a nucleic acid molecule encoding the protein whose amino acid sequence is set forth in SEQ ID NO: 2, and
(v) a nucleic acid molecule whose nucleotide sequence is complementary to the sequence of the nucleic acid molecule of anyone of (i) to (iv).

2. The nucleic acid molecule of claim 1 comprising a nucleic acid molecule whose nucleotide sequence has at least 90% sequence identity with a nucleic acid molecule
(i) whose nucleotide sequence is set forth in SEQ ID NO: 1 or
(ii) which encodes the protein whose amino acid sequence is set forth in SEQ 1D NO: 2.

3. The nucleic acid molecule of claim 1 or 2 comprising a nucleic acid molecule whose nucleotide sequence is set forth in SEQ ID NO: 1, or which encodes the protein whose amino acid sequence is set forth in SEQ ID NO: 2.

4. The nucleic acid molecule of anyone of claims 1 to 3 which encodes a protein that
(i) catalyses terminal and/or subterminal modification of an *n*-alkane substrate having 26 or more carbons,
(ii) carries out oxidation of an *n*-alkane substrate having 26 or more carbons,
(iii) causes the production of alcohols, acids and/or esters from an *n*-alkane substrate having 26 or more carbons,
(iv) is capable of lowering the pour point of a substrate containing an *n-*alkane having 26 or more carbons, or
(v) is capable of complementing a mutation leading to the inactivation of the gene encoded by SEQ ID NO: 1 in *Acinetobacter* sp. DSM17874.

5. The nucleic acid molecule of claim 4 which encodes a protein that causes the production of alcohols, acids and/or esters from an *n*-alkane substrate having 26 or more carbons and
(i) consumes at least 30% of the *n*-alkane substrate, compared to the degree of consumption that is achieved following exposure of the same substrate for the same time under the same conditions to the protein encoded by SEQ ID NO: 1, or
(ii) produces at least 30% of the oxidation product, compared to the amount of product that is achieved following exposure of the same substrate for the same time under the same conditions to the protein encoded by SEQ ID NO: 1.

6. The nucleic acid molecule of claim 4 which encodes a protein that is capable of lowering the pour point of a substrate containing an *n*-alkane having 26 or more carbons and
(i) reduces the pour point of said *n*-alkane containg substrate by at least 30% of the reduction that is achieved following exposure of the same substrate for the same time under the same conditions to the protein encoded by SEQ ID NO: 1, or
(ii) reduces the pour point of said *n*-alkane containing substrate by at least 0.25°C.

7. A fragment of the nucleic acid molecule of any one of claims 1 to 6, that encodes a protein which has the same activity as the full length protein.

8. The fragment of claim 7 which is at least 60 nucleotides in length.

9. The nucleic acid molecule of any one of claims 1 to 8 wherein said *n*-alkane has 32 or more carbons.

10. A protein which has a role in the degradation of an *n*-alkane substrate having 26 or more carbons and which
(i) is encoded by the nucleic acid molecule of any one of claims 1 to 9, or
(ii) comprises a sequence of amino acids selected from the groups consisting of
(a) an amino acid sequence as set forth in SEQ ID NO: 2, or
(b) an amino acid sequence having at least 85% sequence identity with an amino acid sequence as set forth in SEQ ID NO: 2.

11. The protein of claim 10 which has at least 90% sequence identity with an amino acid sequence as set forth in SEQ ID NO: 2.

12. The protein of claim 10 or 11 which has the amino acid sequence as set forth in SEQ ID NO: 2.

13. A functional fragment of the protein of any one of claims 10 to 12, which has the same activity as the full length protein.

14. A chimeric gene comprising the nucleic acid molecule as defined in any one of claims 1 to 9 operably linked to one or more suitable regulatory sequences.

15. A vector comprising the nucleic acid molecule as defined in any one of claims 1 to 9 or the chimeric gene of claim 14

16. A microorganism which has been genetically manipulated to contain a vector of claim 15 and thereby is capable of expressing a protein according to claim 10.

17. A method of causing degradation of an *n*-alkane having 26 or more carbons in a substrate, said method comprising exposing said substrate to a protein that is capable of degrading *n*-alkanes having 26 or more carbon and which comprises a sequence of amino acids selected from the group consisting of
(i) an amino acid sequence as set forth in SEQ ID NO: 2, or
(ii) an amino acid sequence having at least 85% sequence identity with the amino acid sequence as set forth in SEQ ID NO:2, and
(iii) a fragment of (i) or (ii), having the same activity.

18. The method of claim 17 wherein said substrate is crude oil.

19. The method of claim 17 or 18 wherein said protein is as defined in any one of claims 10 to 13.

20. The method of any one of claims 17 to 19 wherein said step of exposure of the substrate to said protein is achieved by contacting the substrate with
(i) a microorganism according to claim 16
(ii) a lysate or extract of a microorganism which is capable of expressing the protein, or
(iii) protein in isolated, extracted or purified form, alone or in combination with one or more other proteins.

21. The method of claim 20 wherein said microorganism is as defined in claim 16.

22. The method of claim 20 wherein said step of exposure of the substrate to said protein is achieved by contacting the substrate with protein in isolated, extracted or purified form, in combination with one or more other proteins and said other protein is selected from a monooxygenase, a rubredoxin, a rubredoxin reductase, an alcohol dehydrogenase, an aldehyde dehydrogenase, an alkane hydroxylase, an acyl coA synthetase and a fatty acid-eoA ligase.

23. A method of decreasing the pour point of an *n*-alkane containing substrate, comprising carrying out the steps as defined in any one of claims 17 to 22.

24. A method of modifying the composition of an *n*-alkane containing substrate comprising carrying out the steps as defined in any one of claims 17 to 22.

## Patentansprüche

1. Nukleinsäuremolekül, das für ein angebliches Monooxygenaseprotein kodiert, das beim Abbau eines *n*-Alkansubstrats, das 26 oder mehr Kohlenstoffatome aufweist, eine Rolle spielt, umfassend eine Nukleinsäuremolekül ausgewählt aus der Gruppe bestehend aus:
(i) einem Nukleinsäuremolekül, dessen Nukleotidsequenz in SEQ ID NO:1 aufgeführt ist,
(ii) einem Nukleinsäuremolekül, dessen Nukleotidsequenz mindestens 80 % Sequenzidentität mit einem Nukleinsäuremolekül aufweist, dessen Nukleotidsequenz in SEQ ID NO:1 aufgeführt ist,
(iii) einem Nukleinsäuremolekül, das für das Protein kodiert, dessen Aminosäuresequenz in SEQ ID NO:2 aufgeführt ist,
(iv) einem Nukleinsäuremolekül, dessen Nukleotidsequenz mindestens 85 % Sequenzidentität mit einem Nukleinsäuremolekül aufweist, das für das Protein kodiert, dessen Aminosäuresequenz in SEQ ID NO:2 aufgeführt ist und
(v) einem Nukleinsäuremolekül, dessen Nukleotidsequenz zur Sequenz des Nukleinsäuremoleküls irgendeines von (i) bis (iv) komplementär ist.

2. Nukleinsäuremolekül nach Anspruch 1, umfassend ein Nukleinsäuremolekül, dessen Nukleotidsequenz mindestens 90 % Sequenzidentität mit einem Nukleinsäuremolekül aufweist
(i) dessen Nukleotidsequenz in SEQ ID NO:1 aufgeführt ist oder
(ii) das für das Protein kodiert, dessen Aminosäuresequenz in SEQ ID NO: 2 aufgeführt ist.

3. Nukleinsäuremolekül nach Anspruch 1 oder 2, umfassend ein Nukleinsäuremolekül, dessen Nukleotidsequenz in SEQ ID NO:1 aufgeführt ist oder das für das Protein kodiert, dessen Aminosäuresequenz in SEQ ID NO: 2 aufgeführt ist.

4. Nukleinsäuremolekül nach einem der Ansprüche 1 bis 3, das für ein Protein kodiert, das
(i) die terminale und/oder subterminale Modifikation eines *n*-Alkansubstrats katalysiert, das 26 oder mehr Kohlenstoffatome aufweist,
(ii) die Oxidation eines *n*-Alkansubstrats, das 26 oder mehr Kohlenstoffatome aufweist, durchführt,
(iii) die Herstellung von Alkoholen, Säuren und/oder Estern aus einem *n-*Alkansubstrat, das 26 oder mehr Kohlenstoffatome aufweist, verursacht,
(iv) in der Lage ist, den Fließpunkt eines Substrats, das ein *n*-Alkan enthält, das 26 oder mehr Kohlenstoffatome aufweist, zu reduzieren oder
(v) in der Lage ist, eine Mutation, die zur Inaktivierung des Gens führt, das durch SEQ NO: 1 kodiert ist, in *Acinetobacter sp.* DSM17874 zu komplementieren.

5. Nukleinsäuremolekül nach Anspruch 4, das für ein Protein kodiert, das die Herstellung von Alkoholen, Säuren und/oder Estern aus einem *n*-Alkansubstrat, das 26 oder mehr Kohlenstoffatome aufweist, verursacht und
(i) mindestens 30 % des *n*-Alkansubstrats im Vergleich mit dem Verbrauchgrad verbraucht, der auf das Aussetzen desselben Substrats für dieselbe Zeitspanne unter denselben Bedingungen wie das Protein, das durch SEQ ID NO:1 kodiert wird, erreicht wird oder
(ii) mindestens 30 % des Oxidationsprodukts im Vergleich mit der Menge an Produkt herstellt, die auf das Aussetzen desselben Substrats für dieselbe Zeitspanne unter denselben Bedingungen wie das Protein, das durch SEQ ID NO:1 kodiert wird, erreicht wird.

6. Nukleinsäuremolekül nach Anspruch 4, das für das Protein kodiert, das in der Lage ist, den Fließpunkt eines Substrats zu reduzieren, das ein *n*-Alkan, das 26 oder mehr Kohlenstoffatome aufweist, enthält und
(i) den Fließpunkt des Substrats, das das *n*-Alkan enthält, um mindestens 30 % der Reduktion reduziert, die auf das Aussetzen desselben Substrats für dieselbe Zeitspanne unter denselben Bedingungen wie das Protein, das durch SEQ ID NO:1 kodiert wird, erreicht wird oder
(ii) den Fließpunkt des Substrats, das das *n*-Alkan enthält, um mindestens 0,25 °C reduziert.

7. Fragment des Nukleinsäuremoleküls nach einem der Ansprüche 1 bis 6, das für ein Protein kodiert, das dieselbe Aktivität wie das Volllängen-Protein aufweist.

8. Fragment nach Anspruch 7, das mindestens 60 Nucleotide lang ist.

9. Nukleinsäuremolekül nach einem der Ansprüche 1 bis 8, wobei das *n*-Alkan 32 oder mehr Kohlenstoffatome aufweist.

10. Protein, das beim Abbau eines *n*-Alkansubstrats, das 26 oder mehr Kohlenstoffatome aufweist, eine Rolle spielt und das
(i) durch das Nukleinsäuremolekül nach einem der Ansprüche 1 bis 9 kodiert wird oder
(ii) eine Sequenz von Aminosäuren umfasst ausgewählt aus der Gruppe bestehend aus
(a) einer Aminosäuresequenz wie in SEQ ID NO: 2 aufgeführt oder
(b) einer Aminosäuresequenz, die mindestens 85 % Sequenzidentität mit einer Aminosäuresequenz wie in SEQ ID NO:2 aufgeführt, aufweist.

11. Protein nach Anspruch 10, das mindestens 90 % Sequenzidentität mit einer Aminosäuresequenz wie in SEQ ID NO:2 aufgeführt, aufweist.

12. Protein nach Anspruch 10 oder 11, das die Aminosäuresequenz wie in SEQ ID NO: 2 aufgeführt, aufweist.

13. Funktionelles Fragment des Proteins nach einem der Ansprüche 10 bis 12, das dieselbe Aktivität wie das Volllängen-Protein aufweist.

14. Chimäres Gen umfassend das Nukleinsäuremolekül wie in einem der Ansprüche 1 bis 9 definiert, das funktionsfähig mit einer oder mehreren geeigneten regulatorischen Sequenzen verknüpft ist.

15. Vektor umfassend das Nukleinsäuremolekül wie in einem der Ansprüche 1 bis 9 definiert oder chimäres Gen nach Anspruch 14.

16. Mikroorganismus, der genmanipuliert worden ist, um einen Vektor nach Anspruch 15 zu enthalten und dadurch in der Lage ist, ein Protein nach Anspruch 10 zu exprimieren.

17. Verfahren zum Verursachen des Abbaus eines *n*-Alkans, das 26 oder mehr Kohlenstoffatome aufweist, in einem Substrat, wobei das Verfahren das Aussetzen des Substrats einem Protein gegenüber umfasst, das in der Lage ist, *n*-Alkane, die 26 oder mehr Kohlenstoffatome aufweisen, abzubauen und das eine Sequenz von Aminosäuren umfasst ausgewählt aus der Gruppe bestehend aus
(i) einer Aminosäuresequenz wie in SEQ ID NO: 2 aufgeführt oder
(ii) einer Aminosäuresequenz, die mindestens 85 % Sequenzidentität mit der Aminosäuresequenz wie in SEQ ID NO: 2 aufgeführt aufweist und
(iii) einem Fragment von (i) oder (ii), die dieselbe Aktivität aufweisen.

18. Verfahren nach Anspruch 17, wobei das Substrat Rohöl list.

19. Verfahren nach Anspruch 17 oder 18, wobei das Protein die Definition wie in einem der Ansprüche 10 bis 13 aufweist.

20. Verfahren nach einem der Ansprüche 17 bis 19, wobei der Schritt des Aussetzens des Substrats dem Protein gegenüber durch Kontaktieren des Substrats mit
(i) einem Mikroorganismus nach Anspruch 16,
(ii) einem Lysat oder Extrakt eines Mikroorganismus, der in der Lage ist, das Protein zu exprimieren oder
(iii) Protein in isolierter, extrahierter oder gereinigter Form als solchem oder in Kombination mit einem oder mehreren anderen Proteinen.

21. Verfahren nach Anspruch 20, wobei der Mikroorganismus die Definition wie in Anspruch 16 aufweist.

22. Verfahren nach Anspruch 20, wobei der Schritt des Aussetzens des Substrats dem Protein gegenüber durch Kontaktieren des Substrats mit Protein in isolierter, extrahierter oder gereinigter Form in Kombination mit einem oder mehreren Proteinen erreicht wird und das andere Protein ausgewählt wird unter einer Monooxygenase, einem Rubredoxin, einer Rubredoxinreduktase, einer Alkoholdehydrogenase, einer Aldehyddehydrogenase, einer Alkanhydroxylase, einer Acyl-CoA-Synthetase und einer Fettsäure-eoA-Ligase.

23. Verfahren zum Reduzieren des Fließpunkts eines *n*-Alkan enthaltenden Substrats umfassend das Durchführen der Schritte wie in einem der Ansprüche 17 bis 22 definiert.

24. Verfahren zum Modifizieren der Zusammensetzung eines *n*-Alkan enthaltenden Substrats umfassend das Durchführen der Schritte wie in einem der Ansprüche 17 bis 22 definiert.

## Revendications

1. Molécule d'acide nucléique qui code pour une protéine monoxygénase putative ayant un rôle dans la dégradation d'un substrat de type *n*-alcane ayant 26 atomes de carbone ou plus, comprenant une molécule d'acide nucléique choisie parmi le groupe constitué de:
(i) une molécule d'acide nucléique dont la séquence nucléotidique est exposée dans SEQ ID n°:1,
(ii) une molécule d'acide nucléique dont la séquence nucléotidique a au moins 80 % d'identité de séquence avec une molécule d'acide nucléique dont la séquence nucléotidique est exposée dans SEQ ID n°:1,
(iii) une molécule d'acide nucléique codant la protéine dont la séquence d'acides aminés est exposée dans SEQ ID n°:2,
(iv) une molécule d'acide nucléique dont la séquence nucléotidique a au moins 85 % d'identité de séquence avec une molécule d'acide nucléique codant la protéine dont la séquence d'acides aminés est exposée dans SEQ ID n°:2, et
(v) une molécule d'acide nucléique dont la séquence nucléotidique est complémentaire de la séquence de la molécule d'acide nucléique selon l'un quelconque des points (i) à (iv).

2. Molécule d'acide nucléique selon la revendication 1, comprenant une molécule d'acide nucléique dont la séquence nucléotidique a au moins 90 % d'identité de séquence avec une molécule d'acide nucléique
(i) dont la séquence nucléotidique est exposée dans SEQ ID n°:1 ou
(ii) qui code pour la protéine dont la séquence d'acides aminés est exposée dans SEQ ID n°:2.

3. Molécule d'acide nucléique selon la revendication 1 ou 2, comprenant une molécule d'acide nucléique dont la séquence nucléotidique est exposée dans SEQ ID n°:1, ou qui code pour la protéine dont la séquence d'acides aminés est exposée dans SEQ ID n°:2.

4. Molécule d'acide nucléique selon l'une quelconque des revendications 1 à 3, qui code pour une protéine qui
(i) catalyse la modification terminale et/ou subterminale d'un substrat de type *n*-alcane ayant 26 atomes de carbone ou plus,
(ii) conduit l'oxydation d'un substrat *n*-alcane ayant 26 atomes de carbone ou plus,
(iii) entraîne la production d'alcools, d'acides et/ou d'esters à partir d'un substrat *n*-alcane ayant 26 atomes de carbone ou plus,
(iv) est capable de réduire le point d'écoulement d'un substrat contenant un *n-*alcane ayant 26 atomes de carbone ou plus, ou
(v) est capable de complémenter une mutation conduisant à l'inactivation du gène codé par SEQ n°:1 dans *Acinetobacter* sp. DSM17874.

5. Molécule d'acide nucléique selon la revendication 4, qui code pour une protéine provoquant la production d'alcools, d'acides et/ou d'esters à partir d'un substrat *n*-alcane ayant 26 atomes de carbone ou plus et
(i) consomme au moins 30 % du substrat de type *n*-alcane, comparé au degré de consommation qui est atteint suite à l'exposition du même substrat sur la même durée sous les mêmes conditions à la protéine codée par SEQ n°:1, ou
(ii) produit au moins 30 % du produit d'oxydation, comparé à la quantité de produit qui est atteinte suite à l'exposition du même substrat sur la même durée sous les mêmes conditions à la protéine codée par SEQ n°:1.

6. Molécule d'acide nucléique selon la revendication 4, qui code pour une protéine étant capable de réduire le point d'écoulement d'un substrat contenant un *n*-alcane ayant 26 atomes de carbone ou plus et
(i) réduit le point d'écoulement dudit substrat contenant un *n*-alcane d'au moins 30 % de la réduction qui est atteinte suite à l'exposition du même substrat sur la même durée aux mêmes conditions à la protéine codée par SEQ ID n°:1, ou
(ii) réduit le point d'écoulement dudit substrat contenant un *n*-alcane d'au moins 0,25°C.

7. Fragment de la molécule d'acide nucléique selon l'une quelconque des revendications 1 à 6, qui code pour une protéine ayant la même activité que la protéine de pleine longueur.

8. Fragment selon la revendication 7, qui est au moins d'une longueur de 60 nucléotides.

9. Molécule d'acide nucléique selon l'une quelconque des revendications 1 à 8, dans laquelle ledit *n*-alcane possède 32 atomes de carbone ou plus.

10. Protéine ayant un rôle dans la dégradation d'un substrat de type *n*-alcane ayant 26 atomes de carbone ou plus et qui
(i) est codée par la molécule d'acide nucléique selon l'une quelconque des revendications 1 à 9, ou
(ii) comprend une séquence d'acides aminés choisie parmi les groupes constitués de
(a) une séquence d'acides aminés telle qu'exposée dans SEQ ID n°:2, ou
(b) une séquence d'acides aminés ayant au moins 85 % d'identité de séquence par rapport à une séquence d'acides aminés telle qu'exposée dans SEQ ID n°:2.

11. Protéine selon la revendication 10, qui a au moins 90 % d'identité de séquence avec une séquence d'acides aminés telle qu'exposée dans SEQ ID n°:2.

12. Protéine selon la revendication 10 ou 11, ayant la séquence d'acides aminés telle qu'exposée dans SEQ ID n°:2.

13. Fragment fonctionnel de la protéine selon l'une quelconque des revendications 10 à 12, ayant la même activité que la protéine de pleine longueur.

14. Gène chimérique comprenant la molécule d'acide nucléique telle que définie selon l'une quelconque des revendications 1 à 9, fonctionnellement lié à une séquence de régulation appropriée ou plus.

15. Vecteur comprenant la molécule d'acide nucléique telle que définie selon l'une quelconque des revendications 1 à 9 ou gène chimérique selon la revendication 14.

16. Micro-organisme qui a été génétiquement manipulé pour contenir un vecteur selon la revendication 15 et qui est ainsi capable d'exprimer une protéine selon la revendication 10.

17. Procédé provoquant la dégradation d'un *n*-alcane ayant 26 atomes de carbone ou plus dans un substrat, ledit procédé comprenant l'exposition dudit substrat à une protéine qui est capable de dégrader les *n*-alcanes ayant 26 atomes de carbone ou plus et comprenant une séquence d'acides aminés choisie parmi le groupe constitué de
(i) une séquence d'acides aminés telle qu'exposée dans SEQ ID n°:2, ou
(ii) une séquence d'acides aminés ayant au moins 85 % d'identité de séquence par rapport à la séquence d'acides aminés telle qu'exposée dans SEQ ID n°:2, et
(iii) un fragment de (i) ou (ii), ayant la même activité.

18. Procédé selon la revendication 17, dans lequel ledit substrat est de l'huile brute.

19. Procédé selon la revendication 17 ou 18, ladite protéine étant telle que définie selon l'une quelconque des revendications 10 à 13.

20. Procédé selon l'une quelconque des revendications 17 à 19, dans lequel ladite étape d'exposition du substrat à ladite protéine est atteinte par la mise en contact du substrat avec
(i) un micro-organisme selon la revendication 16,
(ii) un lysat ou un extrait d'un micro-organisme qui est capable d'exprimer la protéine, ou
(iii) une protéine sous une forme isolée, extraite ou purifiée, seule ou en combinaison avec une ou plusieurs autre(s) protéine(s).

21. Procédé selon la revendication 20, dans lequel ledit micro-organisme est tel que défini dans la revendication 16.

22. Procédé selon la revendication 20, dans lequel ladite étape d'exposition du substrat à ladite protéine est atteinte par mise en contact du substrat avec la protéine sous une forme isolée, extraite ou purifiée, en combinaison avec une ou plusieurs autre(s) protéine(s) et ladite autre protéine est choisie parmi une monoxygénase, une rubrédoxine, une rubrédoxine réductase, une alcool déshydrogénase, une aldéhyde déshydrogénase, une alcane hydroxylase, une acyl-coA synthétase et une acide gras-eoA ligase.

23. Procédé de réduction du point d'écoulement d'un substrat contenant un n-alcane, comprenant la conduite des étapes telles que définies selon l'une quelconque des revendications 17 à 22.

24. Procédé de modification de la composition d'un substrat contenant un n-alcane comprenant la conduite des étapes telles que définies selon l'une quelconque des revendications 17 à 22.
